# EUROPEAN PATENT SPECIFICATION

(11) **EP 1 349 446 B1**
(45) Date of publication and mention of the grant of the patent: **23.01.2013**
(21) Application number: 01999277.5
(22) Date of filing: 07.12.2001
(51) Int. Cl.: A01H 5/00, C12N 15/11, C12N 15/29, C12N 15/54

(54) **MODIFICATION OF SUCROSE SYNTHASE GENE EXPRESSION IN PLANT TISSUE AND USES THEREFOR**
MODIFIKATION DER SACCHAROSESYNTHASE-GENEXPRESSION IN PFLANZENGEWEBE UND IHRE VERWENDUNG
MODIFICATION DE L'EXPRESSION GENETIQUE DE LA SUCROSE SYNTHASE DANS LE TISSU VEGETAL ET SES APPLICATIONS

(30) Priority: 08.12.2000 AU PR197500; 08.12.2000 US 251852 P
(43) Date of publication of application: 08.10.2003
(73) Proprietor: COMMONWEALTH SCIENTIFIC AND INDUSTRIAL RESEARCH ORGANISATION, Campbell, ACT 2601 (AU)
(72) Inventor: LLWELLYN, Danny, O'Connor, Australian Capital Territory 2 (AU); FURBANK, Robert, Weetangara, Australian Capital Territory (AU); RUAN, Yong-Ling, Nicholls, Australian Capital Territory 2 (AU)
(74) Representative: Owen, Deborah Jane
(86) International application number: PCT/AU2001/001580
(87) International publication number: WO 2002/045485

(56) References cited:
- TANG GUO-QING ET AL: "Antisense repression of sucrose synthase in carrot (Daucus carota L.) affects growth rather than sucrose partitioning" PLANT MOLECULAR BIOLOGY, vol. 41, no. 4, November 1999 (1999-11), pages 465-479, XP002327704 ISSN: 0167-4412
- RUAN Y.-L. ET AL.: 'Pathway and control of sucrose import into initiating cotton fiber cells' AUSTRALIAN JOURNAL OF PLANT PHYSIOLOGY vol. 27, 2000, pages 795 - 800, XP002985119
- RUAN Y.-L. AND CHOUREY P.S.: 'A fiberless seed mutation in cotton is associated with lack of fiber cell initiation in ovule epidermis and alterations in sucrose synthase expression and carbon partitioning in developing seeds' PLANT PHYSIOLOGY vol. 118, 1998, pages 399 - 406, XP002984081
- CHENGAPPA S. ET AL.: 'Transgenic tomato plants with decreased sucrose synthase are unaltered in starch and sugar accumulation in the fruit' PLANT MOLECULAR BIOLOGY vol. 40, 1999, pages 213 - 221, XP002984083
- D'AOUST M.-A. ET AL.: 'Antisense inhibition of tomato fruit sucrose synthase decreases fruit setting and the sucrose unloading capacity of young fruit' THE PLANT CELL vol. 11, 1999, pages 2407 - 2418, XP002984084
- RUAN Y.-L. ET AL.: 'The differential expression of sucrose synthase in relation to diverse patterns of carbon partitioning in developing cotton seed' PLANT PHYSIOLOGY vol. 115, 1997, pages 375 - 385, XP002984082
- SHIMIZU Y. ET AL.: 'Changes in levels of mRNAs for cell wall-related enzymes in growing cotton fiber cells' PLANT AND CELL PHYSIOLOGY vol. 38, no. 3, 1997, pages 375 - 378, XP002064957
- HAIGLER C.H. ET AL.: 'Carbon partitioning to cellulose synthesis' PLANT MOLECULAR BIOLOGY vol. 47, 2001, pages 29 - 51, XP002984085

## Description

### Technical Field

The present invention relates to modifying targeted gene expression in plants to obtain a desired effect in the plant.

### Background Art

Sucrose synthase (SuSy) is a key enzyme in the breakdown of sucrose in all plant sink tissue, including grain and fruit and has been extensively studied in many plants. Only relatively recently, however, has this protein and gene been characterised from cotton. The full length (2625 bp) of cotton sucrose synthase (SuSy) was isolated by Perez-Grau, L and Delmer, D in UC, Davis (accession number U73588) in May 1996. A 2030 bp fragment of the same cDNA with 595 bp missing at the 5' end was isolated by the same group in 1994 and was given to Prem Chourey in USDA/ARS for collaborative research. However, no evidence was available at that time regarding the role of this SuSy gene in cotton fibre/seed development, although it had been speculated (Amor *et al.,* 1995) that part of the fibre localised SuSy could associate with cellulose synthase playing a role to channel carbon to this enzyme.

Evidence has been obtained that the expression of the SuSy gene could be important not only for cellulose synthesis but also for fibre cell initiation (thus may control fuzz) and a model on how sucrose is partitioned and competed for between fibre, seed coat and embryos of the cotton seed was proposed (Ruan et al 1997 Plant Physiol, 915, 375-385; Ruan and Chourey 1998 Plant Physiol. 118, 399-406). More recently, the present inventors obtained further evidence supporting the hypothesis that SuSy plays a key role in mobilising sucrose into initiating fibre cells (Ruan et al., 2000 Aust. J. Plant Physiol. 27, 795-800). Tang et al. (1999 Plant Molecular Biol. 41, 465-479) discusses that antisense repression of sucrose synthase in carrots affects growth rather than sucrose partitioning. Chengappa et al. (1999 Plant Molecular Biol. 40, 213-221) describes transgenic tomato plants with decreased sucrose synthase which are unaltered in starch and sugar accumulation in the fruit. D'Aoust et al. (1999 The Plant Cell 11, 2407-2418) discusses antisense inhibition of tomato fruit sucrose synthase decreasing fruit setting and the sucrose unloading capacity of young fruit.

The art is thus deficient in providing methods and means for altering the fibre development and properties in plants, particularly cotton, through alteration of sucrose synthase levels in cells of the plants. The present inventors have now shown that it is possible to modify one or more fibre characteristics and/or fibre content by modifying SuSy gene expression, particularly by modifying SuSy expression in transgenic cotton.

These and other problems are solved as described hereinafter in the different embodiments and claims.

Throughout this specification, unless the context requires otherwise the word "comprise", or variations such as "comprises" or "comprising", will be understood to imply the inclusion of a stated element, integer or step, or group of elements, integers or steps but not the exclusion of any other element, integer or step or group of elements, integers or steps.

### Summary of the invention.

A method for altering fibre development or properties of a fibre producing plant, for example a cotton plant, such as, for example, a cotton plant of the variety fibermax and particularly the Fibermax^{™} variety, may comprise the steps of providing cells of said plants with a chimeric gene comprising the following operably linked DNA fragments: a plant operable promoter, for example a subterranean clover stunt virus promoter; a coding region which when transcribed yields an RNA said RNA being capable of reducing the expression of an endogenous sucrose synthase gene, for example an endogenous sucrose synthase gene expressed in fibre cells, such as fibre initial cells, or capable of being translated into an active sucrose synthase protein; and transcription termination and polyadenylation signals that function in the plant cells.

The present invention provides in particular a method for increasing fibre length in a cotton plant comprising providing cells of said cotton plant with a chimeric gene comprising the following operably linked DNA fragments:
(i) a plant operable promoter;
(ii) a coding region which when transcribed yields an RNA said RNA being capable of being translated into an active sucrose synthase protein; and
(iii) a transcription termination and polyadenylation signal that functions in said plant cells.

Thus, in the method of the invention the RNA is capable of being translated into an active sucrose synthase protein. In a preferred embodiment the coding regions comprise a nucleotide sequence selected from the group consisting of: (a) a nucleotide sequence encoding a polypeptide comprising the amino acid sequence of SEQ ID NO: 2; (b) a nucleotide sequence comprising the nucleotide sequence of SEQ ID NO: 1; (c) a nucleotide sequence having at least 70% sequence identity with the nucleotide sequence of (a) or (b); and (d) a nucleotide sequence hybridizing under stringent conditions with the complementary sequence of (a) or (b); and (e) a fragment of any one of (a) through (d) encoding an active sucrose synthase.

As also described herein, RNA is capable of reducing the expression of an endogenous sucrose synthase gene, for example using coding regions comprising a nucleotide sequence selected from the group consisting of: (a) a nucleotide sequence comprising at least about 19 or 25 contiguous nucleotides having at least 70% sequence identity to a nucleotide sequence encoding a polypeptide comprising the amino acid sequence of SEQ ID NO: 2 or the complement thereof; and (b) a nucleotide sequence comprising at least about 19 or 25 contiguous nucleotides having at least 70% sequence identity from a nucleotide sequence encoding a polypeptide comprising the amino acid sequence of SEQ ID NO: 1 or the complement thereof. More particularly, the coding region comprises the nucleotide sequence of SEQ ID NO: 1 from the nucleotide at about position 2208 to the nucleotide at about position 2598, or the complement thereof. The coding region may comprise simultaneously both sense and antisense nucleotide sequences capable of forming a double stranded RNA molecule.

A method for improving fibre yield in a fibre producing plant may comprise the steps of providing cells of said plant with a chimeric gene comprising the following operably linked DNA fragments: (a) a plant operable promoter; (b) a DNA region capable of being translated into an active sucrose synthase protein; and (c) transcription termination and polyadenylation signals that function in said plant cells.

A method for improving fibre quality in a fibre producing plant may comprise the steps of providing cells of said plant with a chimeric gene comprising the following operably linked DNA fragments: (a) a plant operable promoter; (b) a DNA region capable of being translated into an active sucrose synthase protein; and (c) transcription termination and polyadenylation signals that function in said plant cells.

A method for increasing seed size in a fibre producing plant may comprise the steps of providing cells of said plant with a chimeric gene comprising the following operably linked DNA fragments: (a) a seed-specific promoter; (b) a DNA region capable of being translated into an active sucrose synthase protein; and (c) transcription termination and polyadenylation signals that function in said plant cells.

Fibre producing plants, particularly cotton plants, may comprise in their genome a chimeric DNA comprising the following operably linked DNA fragments: (a) a plant operable promoter; (b) a coding region which when transcribed yields an RNA, said RNA being capable of reducing the expression of an endogenous sucrose synthase gene, e.g. an endogenous sucrose synthase gene expressed in fibre cells, such as fibre initial cells, or alternatively, which is capable of being translated into an active sucrose synthase protein; and (c) transcription termination and polyadenylation signals that function in said plant cells.

A fibre producing plant may comprise a chimeric DNA wherein said DNA is transcribed to yield RNA that is capable of increasing the expression of an endogenous sucrose synthase gene, e.g. an endogenous sucrose synthase gene expressed in fibre cells, such as fibre initial cells and said fibre cells have an increased sucrose synthase activity compared to fibre cells of plant cells which do not comprise said chimeric DNA.

The present invention provides in particular a cotton plant comprising in its genome a chimeric DNA comprising the following operably linked DNA fragments:
(i) a plant operable promoter;
(ii) a coding region which when transcribed yields an RNA said RNA being capable of being translated into an active sucrose synthase protein; and
(iii) a transcription termination and polyadenylation signal that functions in said plant cells.

Another objective of the invention is to provide a cotton plant comprising a chimeric DNA according to the invention, wherein said chimeric DNA is transcribed to yield RNA that is capable of being translated into an active sucrose synthase protein, thereby increasing the level of sucrose synthase in the plant relative to a plant that does not comprise said chimeric DNA. Preferably, the active sucrose synthase is expressed in fibre cells, more preferably fibre initial cells, wherein said fibre cells preferably have higher sucrose synthase activity compared to the fibre cells of a plant which does not comprise the chimeric DNA.

In yet another embodiment of the invention cotton plants are provided comprising a chimeric DNA according to the invention, wherein said coding region comprises a nucleotide sequence selected from the group consisting of: (a) a nucleotide sequence encoding a polypeptide comprising the amino acid sequence of SEQ ID NO: 2; (b) a nucleotide sequence comprising the nucleotide sequence of SEQ ID NO: 1; (c) a nucleotide sequence having at least 70% sequence identity with the nucleotide sequence of (a) or (b); (d) a nucleotide sequence hybridizing under stringent conditions with a complementary sequence of (a) or (b); and (e) a fragment of any one of (a) through (d) encoding an active sucrose synthase.

A fibre producing plant may comprise a chimeric DNA wherein said chimeric DNA encodes an RNA capable of reducing an endogenous sucrose synthase gene expression, such that said fibre cells have a reduced sucrose synthase activity compared to fibre cells of plant cells that do not comprise said chimeric DNA, particularly wherein the coding region comprises a nucleotide sequence selected from the group consisting of: (a) a nucleotide sequence comprising at least about 19 or 25 contiguous nucleotide having at least 70% sequence identity to a nucleotide sequence encoding a polypeptide comprising the amino acid sequence of SEQ ID NO: 2, or the complement thereof; and (b) a nucleotide sequence comprising at least about 19 or 25 contiguous nucleotides having at least 70% sequence identity from a nucleotide sequence encoding a polypeptide comprising the nucleotide sequence of SEQ ID NO: 1 or the complement thereof; and (c) the nucleotide sequence of SEQ ID NO: 1 from the nucleotide at about position 2208 to the nucleotide at about position 2598 or the complement thereof.

Also provided are seeds of the plant of the invention, i.e. the seed of a cotton plant comprising the chimeric DNA or genes according the invention, as well as fibres with altered development or properties, isolated from such plants.

A sucrose synthase encoding nucleotide sequence may be used for altering fibre yield or fibre quality or fibre properties.

### Brief Description of Drawings

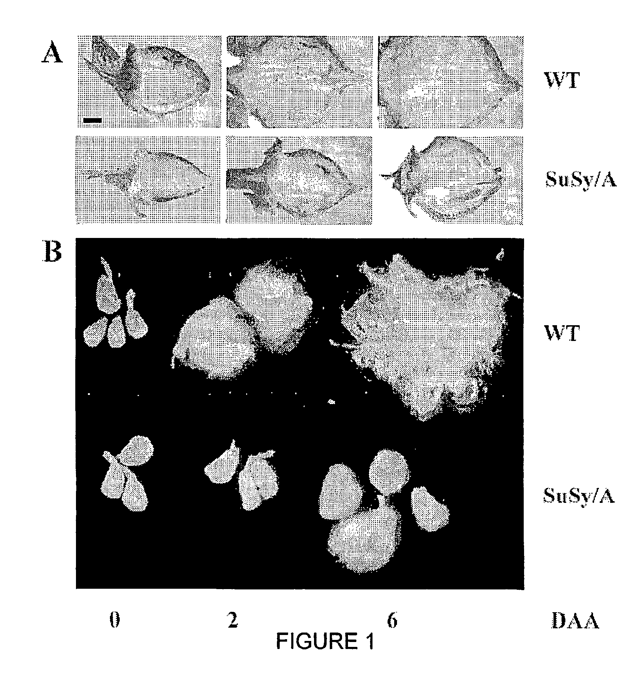

Figure 1 is a photographic representation showing that down regulation of SuSy in the fibre causes a decrease in fibre elongation in transgenic plants. Transverse sections of cotton bolls and seeds of wild type (WT) and transgenic cotton carrying an antisense SuSy gene construct (SuSy/A) are shown. Three developmental stages are shown: 0, 2 and 6 days after anthesis. Data indicate reduced fibre in transgenic lines, particularly in samples 2 days post anthesis (DAA) or older.

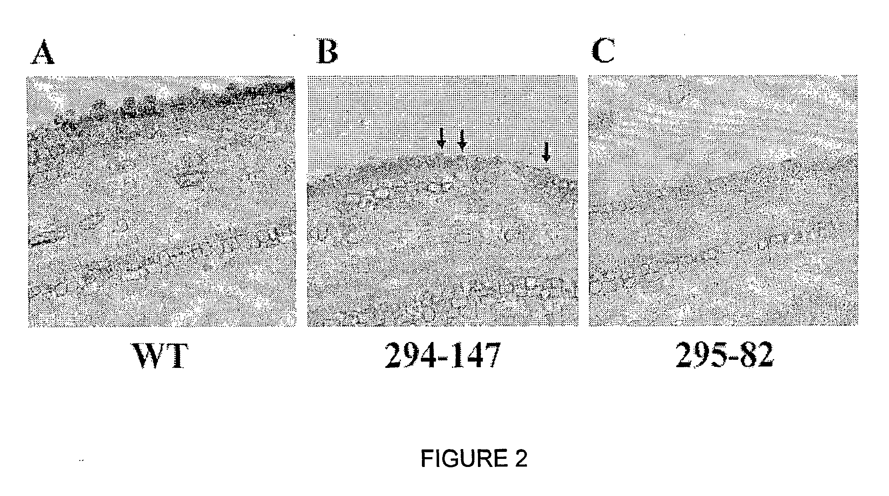

Figure 2 is a photographic representation of an immunohistochemical stain showing localisation of SuSy protein in wild type (WT) cotton (panel A), and the transgenic lines 291-147 (panel B) and 295-82 (panel C). Black coloration in developing fibre cells on the surface of the ovule, and in the ovule epidermis, of the wild type (WT) plants indicates SuSy protein. Transgenic lines show reduced amount of SuSy protein (reduced black coloration). No SuSy protein was detectable in line 295-82. This reduced SuSy protein correlates with lack of fibre development in the transgenic lines.

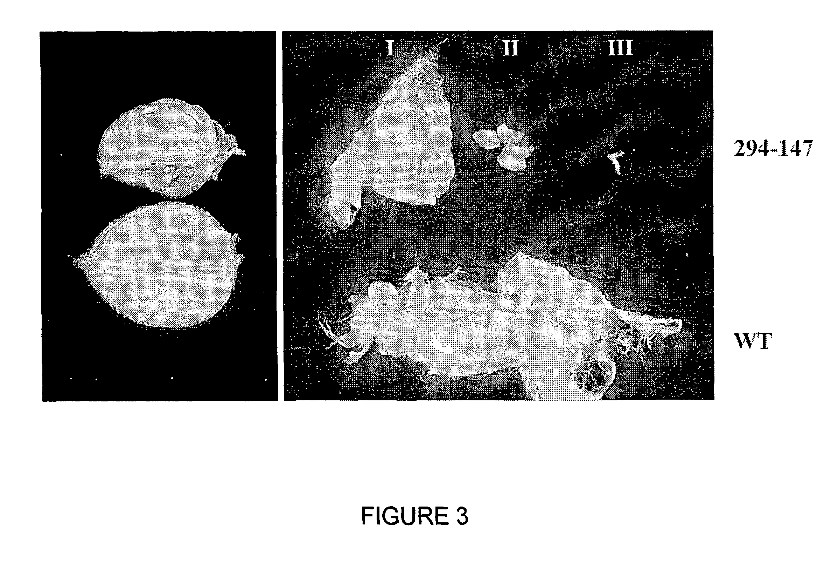

Figure 3 is a photographic representation of cotton bolls (left) and seed (right) from wild type (WT) cotton and the transgenic line 294-147 carrying an antisense SuSy gene construct, which has reduced SuSy protein in the embryo (upper boll and seeds). Seeds from the transgenic line are fibreless, but there is also a great reduction in seed size in seeds labeled III. Some seeds did not form at all.

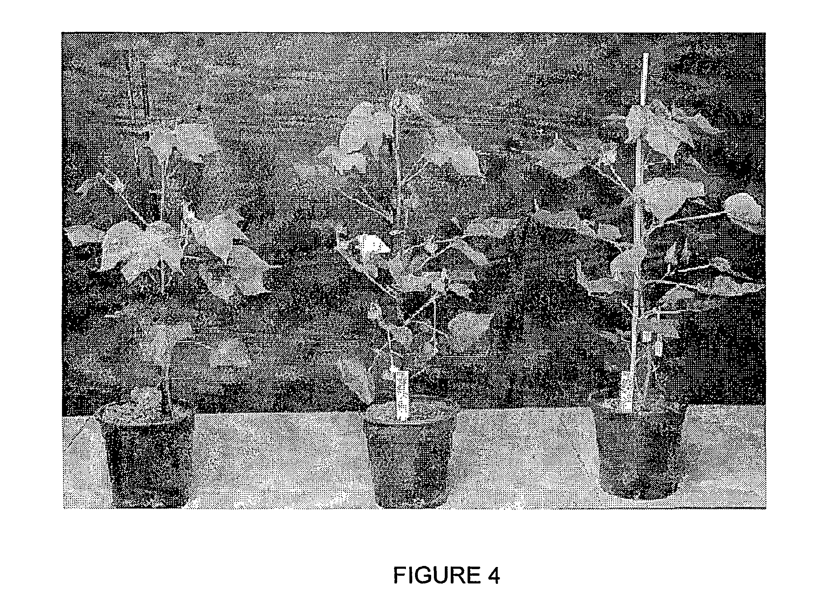

Figure 4 is a photographic representation of wild type cotton plants (left of the figure) and two transformed cotton lines (plants at the middle and right of the figure). Data show that there was no detectable phentotypic effect of the transgene with respect to vegetative growth of plants.

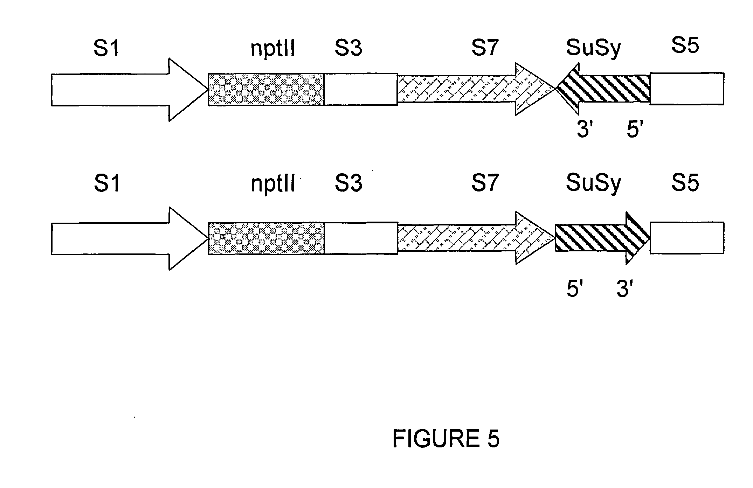

Figure 5 is a schematic representation of an antisense gene construct (top) and a cosuppression gene construct (below) used to suppress SuSy gene expression *in planta.* Gene construct components are as follows: nptII, neomycin phosphotransferase marker gene for selection of transformants; S1 and S7, subclover stunt virus promoters 1 and 7, respectively (see Australian Patent No 689311); S3 and S5, subclover stunt virus gene terminator sequences 3 and 5, respectively (see Australian Patent No 689311); SuSy, a 390 bp nucleotide sequence of a sucrose synthase-encoding cDNA (Genbank Accession number U73588) from CTGGGAT to TGACTT (ie. 211 bp coding region upstream of the stop codon plus 179 bp 3'-untrnaslated (UTR) region; nucleotide position 2208 to nucleotide position 2598 of SEQ ID NO: 1). Arrows indicate the orientation of fragments relative to their native orientation in the genes from which they were derived.

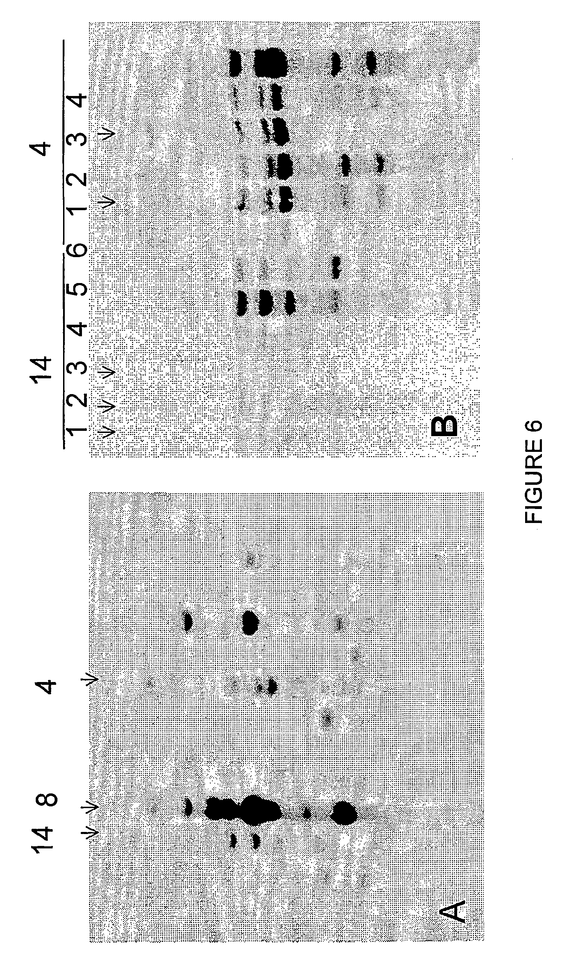

Figure 6 is a copy of a photographic representation of a Southern blot hybridization, showing SuSy gene copy in wild type cotton plants and transgenic cotton lines. DNAs were isolated from T0 generation plants (panel A), and a segregating population of T1 generation plants from the primary transformed lines designated 14 and 4 (panel B). The arrows indicate lines 14, 8, and 4 in the T0 generation having a fibre-less or reduced fibre phenotype (panel A), and lines 14-1, 14-2, 14-3, 4-1, and 4-3 in the segregating population of T1 plants having a fibre-less or reduced fibre phenotype (panel B). The numbers indicate the transgenic cotton line from which the individual plants were derived.

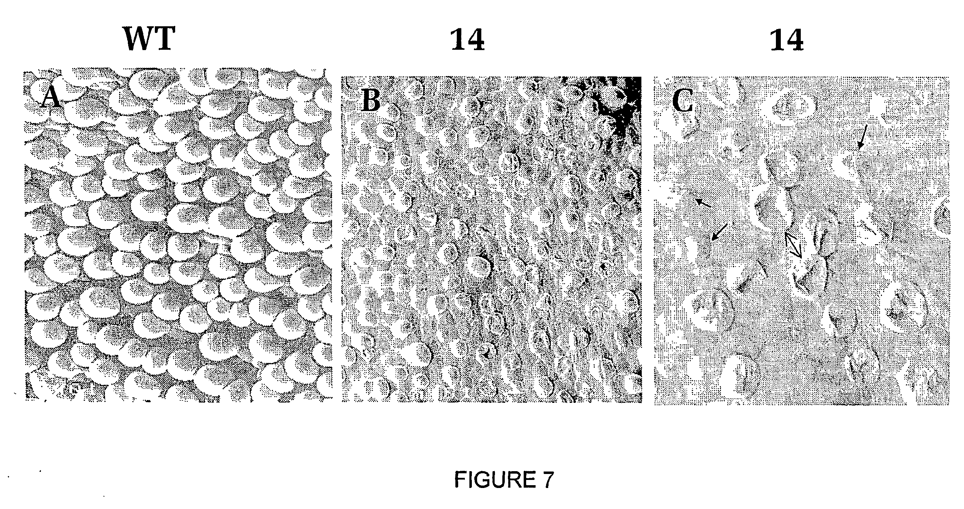

Figure 7 is a photographic representation of a scanning electron microscopy (SEM) profile of the ovule epidermis of wild type cotton (panel A) and line 294-147 shown in Fig. 2B (panels B and C). Many fibre cells are collapsed and/or shrunken in the transgenic line, compared to the wild type plants. Whilst some fibre cells appeared in the transgenic lines, these were much smaller in size and fewer in number than in the wild type ovule (compare panels A and B). The shrunken and/or collapsed phenotype of the trasngenic line was clearly demonstrated under high magnification (panel C).

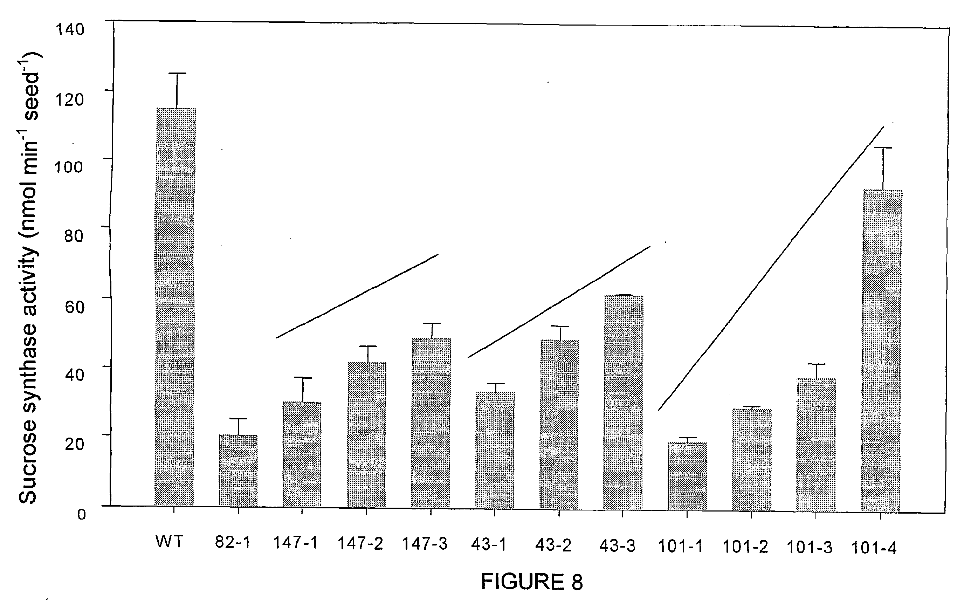

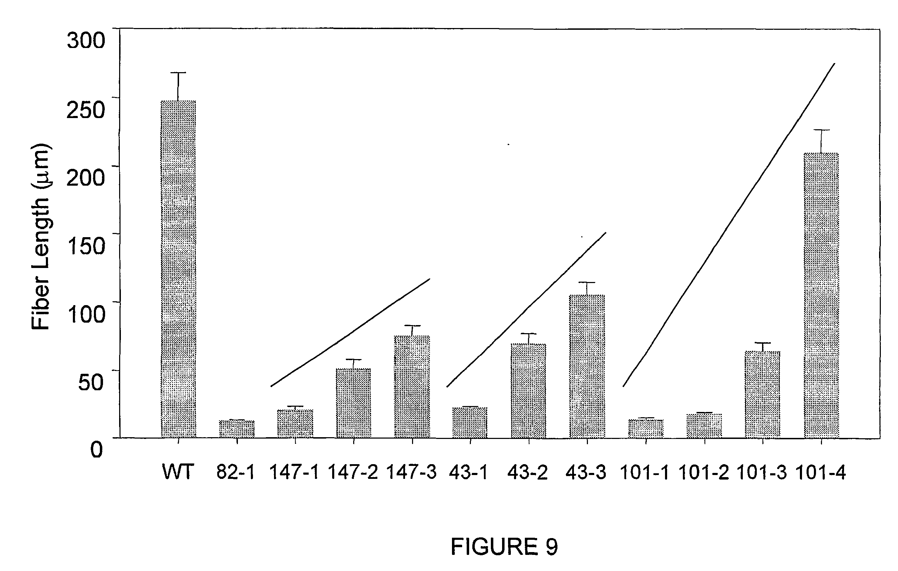

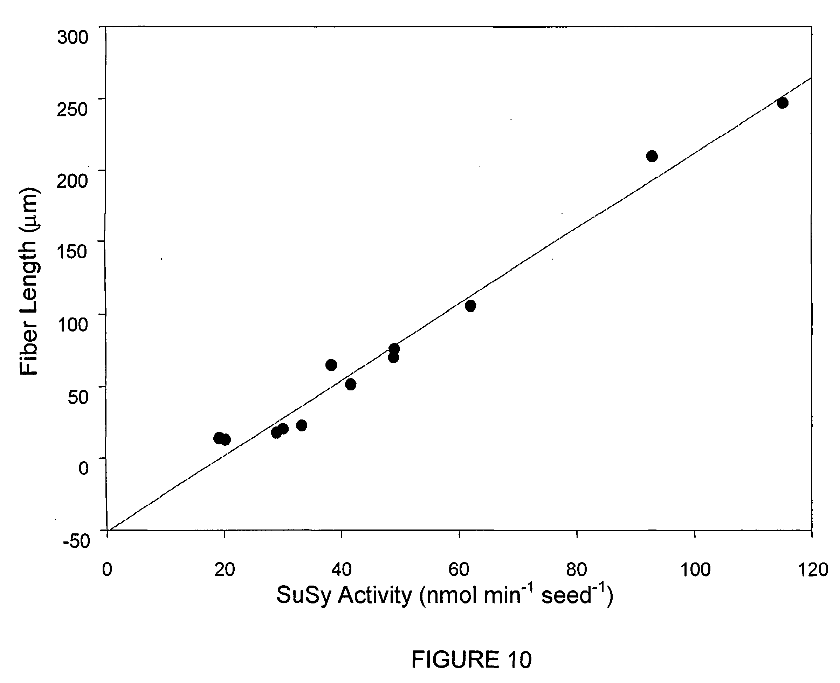

Figures 8, 9 and 10 shows the correlation between changes of SuSy activity (Fig. 8) and reduction of fibre length (Fig. 9) in 3 day old seeds from 10 segregating individuals of three T1 generation lines (lines 147, 43 and 101), together with line 82 and wild type (WT). Figure 10 represents the regression analysis between SuSy activity and fibre length.
Figure 8 is a graphical representation showing modified SuSy activity (abscissa) in transgenic lines compared to wild type plants. Plant lines are indicated on the x-axis. Error bars represent the SEM for assays of 3-day old seeds from 10 segregating individuals of the T1 generation lines.
Figure 9 is a graphical representation showing modified fibre length (abscissa) in transgenic lines compared to wild type plants. Plant lines are indicated on the x-axis. Error bars represent the SEM for assays of 3-day old seeds from 10 segregating individuals of the T1 generation lines.
Figure 10 is a graphical representation of a regression analysis between SuSy activity data represented in Figure 8 (x-axis) and fibre length data presented in Figure 9 (abscissa). Data show a strong correlation (R2=0. 98) between SuSy activity and fibre length over the range shown.

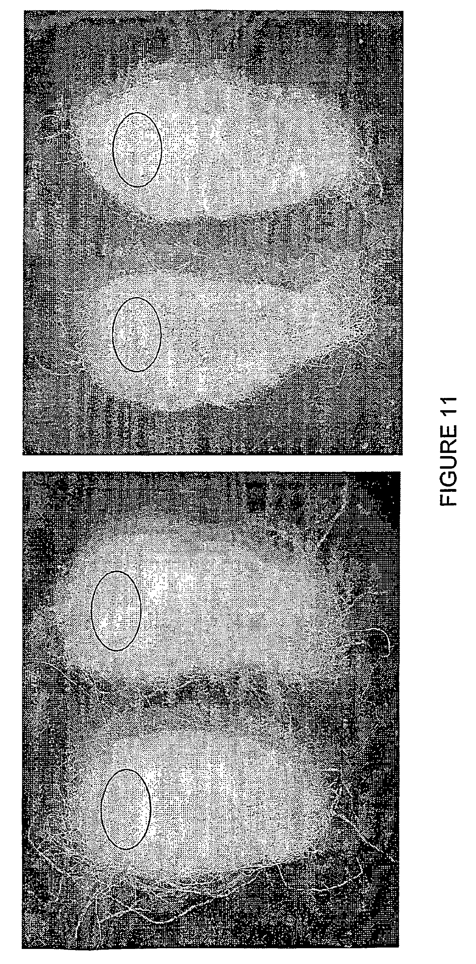

Figure 11 is a photographic representation showing fuzz fibre length in seeds of wild type cotton (panel A) and the transgenic line 147#2 (panel B), and showing reduced fuzz in the transgenic line. Data demonstrate that a reduction in SuSy gene expression reduces fuzz. Seed were delinted by hand. The oval represents the chalazal end of the mature seeds. Seed from the transgenic line are more brown in appearance than wild type seeds, because the underlying brown seed coat is covered by shorter fuzz fiber than in wild type seed.

### Detailed Description of the Invention

A plant having altered expression of an iso-form of sucrose synthase (SuSy) may have an altered fibre, fruit or seed production ability.

The plant may be a transgenic plant having an under-or overexpression of an iso-form of SuSy involved in fruit/seed production, for example the plant retains expression of SuSy in developing vegetative tissue such that growth and development of the plant is not adversely affected.

When the expression of an isoform of SuSy that is involved in fruit/seed production is reduced in the plant, for example in the seed, e.g. in the fibre producing cells, such as fibre initial cells, the plant has a reduced fibre and/or seed production. When the expression of an isoform of SuSy that is involved in fruit/seed production is enhanced (i. e. SuSy is overexpressed) in the plant, for example in the seed, e.g. in the fibre cells, such as fibre initial cells, the plant has enhanced fibre and/or seed production.

The ability to cause underexpression of an iso-form of SuSy involved in fruit/seed production can be used to develop plants which produce few or no seeds but are still able to grow and produce fruit normally. The ability to cause overexpression of an iso-form of SuSy involved in fruit/seed production in a tissue specific manner can be used to develop plants which produce greater amounts of fibre or longer fibre or having altered fibre structure but are still able to grow and develop normally. The plant can be any plant in which the expression of an iso-form of SuSy is involved in fruit/seed production. The method of present invention as described herein is applicable for modifying a wide range of horticultural crops such as grape, peach, pear, and apple. In the present invention, the plant is a cotton plant.

Plants, particularly cotton plants, may be provided comprising a chimeric gene, which may be stably integrated in their genome, the chimeric gene comprising the following operably linked DNA fragments:
a) a plant operable promoter
b) a coding region which when transcribed yields an RNA which is either
   i) capable of reducing the expression of an endogenous sucrose synthase gene, preferably an endogenous sucrose synthase gene expressed in fibre initial cells; or
   ii) capable of being translated into an active sucrose synthase protein; and
c) transcription termination and polyadenylation signals that function in plant cells.

The present invention provides in particular a cotton plant comprising in its genome a chimeric DNA comprising the following operably linked DNA fragments:
i. a plant operable promoter;
ii. a coding region which when transcribed yields an RNA said RNA being capable of being translated into an active sucrose synthase protein; and
iii. a transcription termination and polyadenylation signal that functions in said plant cells.

A particularly preferred embodiment of the coding region as defined in sub ii) or b) above is a coding region comprising a nucleotide sequence which encodes a protein comprising the amino acid of SEQ ID NO: 2, particularly the nucleotide sequence of SEQ ID NO: 1, or a fragment of such a nucleotide sequence capable of being translated into a functional sucrose synthase. The coding region encoding the sucrose synthase activity may be derived from other plant species or other species (as indicated elsewhere in this application), such as, for example, from potato (cDNA sequence is available from Genbank library, accession number M18745).

The coding region as defined in sub b) i) may be a coding region comprising the complement of a nucleotide sequence ("antisense") encoding a polypeptide encoded by the nucleotide sequence of SEQ ID NO : 1 from the nucleotide at about position 2208 to the nucleotide at about position 2598, particularly the coding region comprises the nucleotide sequence of SEQ ID NO: 1 from the nucleotide at position 2208 to the nucleotide at position 2598.

As used herein, the term"coding region"shall be taken to mean a DNA sequence which is capable of being transcribed into a biologically active RNA, irrespective of whether or not said DNA sequence encodes an amino acid sequence. In the present context, a biologically active RNA includes RNA which is capable of inducing a biological effect in the target cell, such as antisense or sense RNA capable of triggering post transcriptional gene silencing, or a ribozyme etc.

Biologically active RNA also includes RNA which is capable of being translated into a polypeptide or protein.

Having read these preferred embodiments, the person skilled in the art will immediately realize that functional equivalents of the mentioned coding regions may be used to similar effect.

Functional equivalents of a coding region capable of being transcribed into RNA which is capable of reducing the expression of an endogenous sucrose synthase gene, include e. g. shorter antisense fragments compared to the above-mentioned nucleotide sequences.

The length of the antisense nucleotide sequence may vary from about 10 nucleotides (nt) or 19 nt, up to a length equaling the length (in nucleotides) of the target nucleic acid (i. e. the full-length sucrose synthase gene). In some cases, the total length of the antisense nucleotide sequence is at least 10 nt, 15 nt or 19 nt, or at least about 50 nt, at least about 100 nt, at least about 150 nt, at least about 200 nt, or at least about 500 nt. It is expected that there is no upper limit to the total length of the antisense nucleotide sequence, other than the total length of the target nucleic acid. However for practical reasons (such as e.g. stability of the chimeric genes) it is expected that the length of the antisense nucleotide sequence should not exceed 5000 nt, e.g. should not exceed 2500 nt. Conveniently, the length of an antisense nucleic acid is limited to about 1000 nt.

It will be appreciated that the longer the total length of the antisense nucleotide sequence is, the less stringent the requirements for sequence identity between the total antisense nucleotide sequence and the complement of the corresponding sequence in the target sucrose synthase gene become.

The total antisense nucleotide sequence should have a sequence identity of at least about 75% with the complement corresponding target sequence, e.g. at least about 80 %, 85%, 90%, 95% or, 100%, or be identical to complement of the corresponding part of the target nucleic acid. In some cases the antisense nucleotide sequence always includes a sequence of about 10 consecutive nucleotides, e.g. about 19 or 20 nt, about 50 nt, about 100 nt, or about 150 nt with 100% sequence identity to the complement of the corresponding part of the target nucleic acid. For calculating the sequence identity and designing the corresponding antisense sequence, the number of gaps should be minimized, particularly for the shorter antisense sequences.

Antisense nucleotide sequences may have a sequence identity of at least about 75%, at least about 80%; at least about 85%; about 90%, or about 95% with the complement of the corresponding part of the nucleotide sequence of SEQ ID NO : 1.

For the purpose of this invention, the "sequence identity" of two related nucleotide or amino acid sequences, expressed as a percentage, refers to the number of positions in the two optimally aligned sequences which have identical residues (x100) divided by the number of positions compared. A gap, i.e. a position in an alignment where a residue is present in one sequence but not in the other is regarded as a position with non-identical residues. The alignment of the two sequences is performed by the Needleman and Wunsch algorithm (Needleman and Wunsch 1970) Computer-assisted sequence alignment, can be conveniently performed using standard software program such as GAP which is part of the Wisconsin Package Version 10.1 (Genetics Computer Group, Madison, Wisconsin, USA) using the default scoring matrix with a gap creation penalty of 50 and a gap extension penalty of 3.

It goes without saying that sense fragments may also be used for reducing the expression of an endogenous sucrose synthase gene, and the same embodiments of length and sequence homology as herein described for antisense molecules, apply mutatis mutandis to the sense molecules.

Particularly suited for reducing the expression of an endogenous sucrose synthase genes are DNA regions, for example under the control of a plant operable promoter, which when transcribed result in so-called double stranded RNA molecules, comprising both sense and antisense sequences which are capable of forming a double stranded RNA molecule as described in WO 99/53050. In this particular case, a chimeric gene may thus be introduced into a plant cell comprising a plant operable promoter operably linked to a DNA region, whereby that DNA region comprises a part of coding region comprising at least 10 or 19 consecutive nucleotides from the coding region of a nucleic acid encoding a sucrose synthase protein, such as but not limited to, a sucrose synthase protein with the amino acid sequence of SEQ ID NO: 1 (the so-called sense part) as well as a DNA sequence which comprises at least the complementary DNA sequence of at least 10 or 19 nucleotides of the sense part, but which may be completely complementary to the sense part (the so-called antisense part). The chimeric gene may comprise additional regions, such as a transcription termination and polyadenylation region functional in plants. When transcribed an RNA can be produced which may form a double stranded RNA stem between the complementary parts of the sense and antisense region. A spacer region may be present between the sense and antisense nucleotide sequence. The chimeric gene may further comprise an intron sequence, e.g. located in the spacer region.

Functional equivalents of coding regions capable of being transcribed into RNA which can be translated into an active sucrose synthase comprise mutant or allelic forms derived from sucrose synthase genes, particularly from sucrose synthase gene with active expression in fibre initials.

Methods to derive mutants e.g. of a sucrose synthase gene, particularly of sucrose synthase genes encoding a protein as represented in SEQ ID NO: 2, quite particularly of sucrose synthase gene comprising the nucleotide sequence of SEQ ID NO: 1, such a site-specific mutagenesis methods are well known in the art, as well as assays to identify active sucrose synthase enzymes encoded by the mutant sequences.

Allelic variants of the nucleotide sequences encoding sucrose synthase may be identified by hybridization of libraries, under stringent conditions, such as cDNA or genomic libraries of a different varieties or plant lines, particularly cotton varieties and plant lines. Nucleotide sequences which hybridize under stringent conditions to nucleotide sequences encoding the amino acid sequence of SEQ ID NO: 2 or to the nucleotide sequence of SEQ ID NO: 1, or a sufficiently large part thereof (preferably about 25 contiguous nucleotides, particularly at least about 50 contiguous nucleotides, more particularly at least about 100 contiguous nucleotides) and which encode a functional protein with sucrose synthase activity are functional equivalents of the above mentioned preferred coding regions. Such nucleotides may also be identified and isolated using e.g. polymerase chain reaction amplification using an appropriate pair of oligonucleotides having at least about 25 contiguous nucleotides, particularly at least about 50 contiguous nucleotides, more particularly at least about 100 contiguous nucleotides of the nucleotide of SEQ ID NO: 1.

"Stringent hybridization conditions" as used herein mean that hybridization will generally occur if there is at least 95% and preferably at least 97% sequence identity between the probe and the target sequence. Examples of stringent hybridization conditions are overnight incubation in a solution comprising 50% formamide, 5 x SSC (150 mM NaCl, 15 mM trisodium citrate), 50 mM sodium phosphate (pH 7.6), 5x Denhardt's solution, 10% dextran sulfate, and 20 µg/ml denatured, sheared carrier DNA such as salmon sperm DNA, followed by washing the hybridization support in 0.1 x SSC at approximately 65 °C. Other hybridization and wash conditions are well known and are exemplified in Sambrook *et al,* Molecular Cloning: A Laboratory Manual, Second Edition, Cold Spring Harbor, NY (1989), particularly chapter 11.

The following are accession numbers of nucleotide sequences in the Genbank library which are plant sucrose synthase genes, parts thereof or nucleotide sequences having sequence similarity to sucrose synthase genes which could be used according to the methods herein described: BM094593 (Glycine max), BM093753 (Glycine max) BM093158 (Glycine max) BM092695(Glycine max) BM092443(Glycine max) BM092322(Glycine max) BM085310(Glycine max) BM085020 (Glycine max) AY059416 (Zea mays) AF273253(Beta vulgaris) L39940 (Oryza sativa) AJ316590 (Nostoc punctiforme) AJ316589 (Nostoc punctiforme) AJ316596 (Anabaena sp) AJ316595(Anabaena sp) AJ316584 (Anabaena sp) BM005654 (Crocus sativus) B1973032 (Glycine max) B1971794 (Glycine max) AF367450 (Prunus persica) B1945506 (Glycine max) B1944973 (Glycine max) AF420224 (Carica papaya) B1788449 (Glycine max) B1788359 (Glycine max) B1787127 (Glycine max) 81787033(Glycine max) B1787000 (Glycine max) 81786823(Glycine max) B1784933 (Glycine max) B1784627 (Glycine max) B1700214 (Glycine max) B1699934 (Glycine max) B1699923 (Glycine max) 81699585(Glycine max) B1543240 (Sugar beet) B1498340 (Glycine max) B1471463 (Glycine max) B1427241 (Glycine max) B1427174 (Glycine max) B1427022 (Glycine max) B1426915 (Glycine max )AF393809 (Apium graveolens) B1321173 (Glycine max) 81320832(Glycine max) B1316894 (Glycine max) B1316826 (Glycine max) B1316405 (Glycine max) B1315949 (Glycine max) B1203222 (Lycopersicon esculentum) B1176503 (Solanum tuberosum) BG273882 (Grape berries) AY034958 (Arabidopsis thaliana) AF378187(Oryza sativa) BG790580 (Glycine max) BG790079 (Glycine max) BG726150 (Glycine max) BG654021 (Glycine max) BG653916 (Glycine max) BG653624 (Glycine max) BG652711 (Glycine max) BG652507 (Glycine max) BG649914 (Glycine max) BG649831 (Glycine max) AJ309093 (Pinus pinaster) BG507656 (Glycine max) BG405632 (Glycine max) BG405474 (Glycine max) BG405204 (Glycine max) BG405017 (Glycine max) BG363730 (Glycine max) BG362638 (Glycine max) BG359764 (Glycine max) BG359452 (Glycine max) BG359307 (Glycine max) AJ311496 (Pisum sativum) BG239317 (Glycine max) BG237287 (Glycine max) BG157592 (Glycine max) BG155900 (Glycine max) BG047402 (Glycine max) BG046717 (Glycine max) BG046686 (Glycine max) BG046043 (Glycine max) BG043243 (Glycine max) BG042159 (Glycine max) BG041814 (Glycine max) AB045710 (Pyrus pyrifolia) BF597682 (Glycine max) BF597330 (Glycine max) BF597258 (Glycine max) BF595837 (Glycine max) BF595646 (Glycine max) BF425609 (Glycine max) BF423725 (Glycine max) BF324657 (Glycine max) BF154308 (Solanum tuberosum) BF154187 (Solanum tuberosum) BF154037 (Solanum tuberosum) BF153500 (Solanum tuberosum) BF153341 (Solanum tuberosum) BF153335 (Solanum tuberosum) BF153307 (Solanum tuberosum) BF097021 (Lycopersicon esculentum) BF070782 (Glycine max) BF070666 (Glycine max) BF070277 (Glycine max) BF070188 (Glycine max) BF068815 (Glycine max) BF071174 (Glycine max) BF067110 (Glycine max) BF009756 (Glycine max) BE806119 (Glycine max) BE805996 (Glycine max) BE805178 (Glycine max) BE800976 (Glycine max) BE800941(Glycine max) BE800227 (Glycine max) BE611805 (Glycine max) BE611493 (Glycine max) BE610447 (Glycine max) BE610325 (Glycine max) BE609887(Glycine max) BE609881 (Glycine max) BE607382 (Oryza sativa) BE607326 (Oryza sativa) BE607323 (Oryza sativa) BE556484 (Glycine max) BE556306 (Glycine max) BE555319 (Glycine max) AJ001117 (Triticum aestivum) AJ292758 (Anabaena variabilis) BE474178 (Glycine max) BE440931 (Glycine max) BE440323 (Glycine max) BE347277 (Glycine max) AF263384 (Saccharum officinarum) BE330345 (Glycine max) BE211050 (Glycine max) BE209707 (Glycine max) AW561929 (Gossypium hirsutum) BE040389 (Oryza sativa) BE040121 (Oryza sativa) BE039563 (Arabidopsis thaliana) BE034387 (Mesembryanthemum crystallinum) BE033387 (Mesembryanthemum crystallinum) BE023957 (Glycine max) BE023630 (Glycine max) BE022760 (Glycine max) BE021167 (Glycine max) BE020760 (Glycine max) BE020591 (Glycine max) BE020550 (Glycine max) AW990923 (Euphorbia esula) AW832022 (Glycine max) AW760552 (Glycine max) AW756812 (Glycine max) AW756072 (Glycine max) AW756065 (Glycine max) AW734901(Glycine max) AW707163 (Glycine max) AW706660 (Glycine max) AW706520 (Glycine max) AW706203 (Glycine max) AW705595 (Glycine max) AW704619 (Glycine max) AB025778 (Citrus unshiu) AB021745 (Citrus unshiu) AW666333 (Glycine max) AW666277 (Glycine max) AW666250 (Glycine max) AW620859 (Glycine max) AW598476 (Glycine max) AW598473 (Glycine max) AW597690 (Glycine max) AW597373 (Glycine max) AW597332 (Glycine max) AW596993 (Glycine max) AW570615 (Glycine max) AW570577 (Glycine max) AW570566 (Glycine max) AW570513 (Glycine max) AW569822 (Glycine max) AW568526 (Glycine max) AW568333 (Glycine max) AW509231 (Glycine max) AW472408 (Glycine max) AW459606 (Glycine max) AW458318 (Glycine max) X82504 (C.rubrum) AW432731(Glycine max) AW432392 (Glycine max) AW432192 (Glycine max) AW397142 (Glycine max) AW397071 (Glycine max) AW309503 (Glycine max) AW307502 (Glycine max) AW307391 (Glycine max)AW307001 (Glycine max) AW306834 (Glycine max) AB018561 (Citrullus lanatus) AB029401 (Citrus unshiu) AB022092 (Citrus unshiu) AB022091 (Citrus unshiu) AW279073 (Glycine max) AW279053 (Glycine max) AW278487 (Glycine max) AJ388994 (Medicago truncatula) AJ388888 (Medicago truncatula) AW234887 (Glycine max) AJ238219 (Triticum aestivum) AJ238218 (Triticum aestivum) AJ238217 (Triticum speltoides) AW201670 (Glycine max) AW185801(Glycine max) AW185627 (Glycine max) AJ249624 (Triticum aestivum) AJ249623 (Triticum aestivum) AW164630 (Glycine max) AW164393 (Glycine max) AW133248 (Glycine max) AW101578 (Glycine max) AW100191 (Glycine max) AW100069 (Glycine max) AW099557 (Glycine max) X96938 (T.gesneriana) X96939 (T.gesneriana) AW035186 (Lycopersicon esculentum) AW033439 (Lycopersicon esculentum) AW032339 (Lycopersicon esculentum) AJ132002 (Craterostigma plantagineum) AJ132001 (Craterostigma plantagineum) AJ132000 (Craterostigma plantagineum) AJ131999 (Craterostigma plantagineum) AI973811 (Glycine max) AI973710 (Glycine max) AI973540 (Glycine max) AI967739 (Lotus japonicus) AI965972 (Glycine max) AI960742 (Glycine max) AI960703 (Glycine max) AI930917 (Glycine max) AI900130 (Glycine max) AI900087 (Glycine max) AI855470 (Glycine max) AA080634 (Saccharum sp.) AA080610 (Saccharum sp.) AA269294 (Saccharum sp.) AA080580 (Saccharum sp.) AI736370 (Glycine max) AI731292 (Gossypium hirsutum) AI731115 (Gossypium hirsutum) AI729201 (Gossypium hirsutum) AI728436 (Gossypium hirsutum) AI27966 (Gossypium hirsutum) AI726092 (Gossypium hirsutum) U73588 (Gossypium hirsutum) U73587 (Gossypium hirsutum) AJ012080 (Pisum sativum) AJ131964 (Medicago truncatula) AJ131943 (Medicago truncatula) AJ133726 (Lotus japonicus) Y16091 (Daucus carota) Y16090 (Daucus carota) AJ011319 (Lycopersicum esculentum) AI496671 (Glycine max) AI496540 (Glycine max) AI496532 (Glycine max) AI495774 (Glycine max) AI495135 (Glycine max) AI495023 (Glycine max) AI494833 (Glycine max) AJ011534 (Lycopersicon esculentum) Y15802 (Hordeum vulgare) AI461126(Glycine max) AI460757 (Glycine max) AI460629 (Glycine max) AI444096 (Glycine max) AI444083 (Glycine max) AI444054 (Glycine max) AI443620 (Glycine max) AI443476 (Glycine max) AI443231 (Glycine max) AI442789 (Glycine max) AI442411 (Glycine max) AI441989 (Glycine max) AI441004 (Glycine max) AI437923 (Glycine max) AI437907 (Glycine max) AI437840 (Glycine max) AJ010639 (Anabaena sp.) AJ011535 (Lycopersicon esculentum) D10266 (Vigna radiata) L03366 (Oryza sativa) AF030231 (Glycine max) M97551 (Vicia faba) AJ000153 (Triticum aestivum) AF079523 (Musa acuminata) AF079851 (Pisum sativum) AJ001071 (Pisum sativum) AF049487 (Medicago sativa) AF054446 (Mesembryanthemum crystallinum) AA753339 (Oryza sativa) AA752298 (Oryza sativa) AA752293 (Oryza sativa) AA753445 (Oryza sativa) AA753437 (Oryza sativa) AA753297 (Oryza sativa) AA752123 (Oryza sativa) AA751990 (Oryza sativa) AA750692 (Oryza sativa) AA750079 (Oryza sativa)AA749692 (Oryza sativa) AA749554 (Oryza sativa) AA720478 (Mesembryanthemum crystallinum) AA661050 (Medicago truncatula) AA661041 (Medicago truncatula) AA660686 (Medicago truncatula) D88412 (Cotton) D10418 (Rice) D21308 (Rice) D29733 (Rice) X81974 (B.vulgaris) X92378 (A.glutinosa) Z56278 (V.faba) Z48640 (V.faba) X98598 (P.sativum) T25261 (Zea mays) T23326 (Zea mays) T14713 (Zea mays) T14662 (Zea mays) T14661 (Zea mays) X75332 (D.carota) X02382 (Zea mays) X02400 (Zea mays) X70990 (A.thaliana) X60987 (A.thaliana) X69773 (V. faba) X73477 (S. tuberosum) Z11532 (S. officinarum) Z15028 (O. sativa) X64770 (O. sativa) X59046 (O. sativa) X66728 (H. vulgare) X65871 (H. vulgare) X69931 (H. vulgare) A27685 (O. sativa) W21612 (Zea mays) U24088 (Solanum tuberosum) U24087 (Solanum tuberosum) X73221 (H. vulgare) L32898 (Zea mays) F13913 (Arabidopsis thaliana) F13912 (Arabidopsis thaliana) U21129 (Solanum tuberosum) M26672 (Triticum aestivum) M26671 (Triticum aestivum) L19762 (Lycopersicon esculentum) M18745 (Potato) L33244 (Zea mays) L22296 (Zea mays) Z17959 (Arabidopsis thaliana). These sequences are incorporated by reference.

A method of altering the production of fibre, fruit or seeds in a plant, may comprise causing under- or overexpression of an iso-form of SuSy involved in fruit/seed production in the plant.

For underexpression, the method may involve providing a genetic construct which targets the 3' end of the SuSy gene when transformed in a plant. By "3'-end" is meant a sequence encoding the C-terminal portion of a SuSy polypeptide with or without the 3' non-coding sequence, or a sequence that is complementary thereto. The construct can be a co-suppression antisense or combined sense/antisense (inverted repeat) SuSy construct.

A plant having an altered ability to produce fibre, fruit or seeds may be produced by the method described above.

A genetic construct targeting the 3'end of the SuSy gene may, when expressed in a plant, reduces the expression of an iso-form of SuSy involved in fruit/seed production.

The chimeric genes, particularly of the coding regions used for these methods are as described elsewhere in this specification.

The construct may be selected from the constructs shown in Figure 5, a combination of parts of both constructs to generate an inverted repeat gene suppression construct, with or without a tissue specific promoter region.

One method of developing constructs is by using the"pPLEX" technology involving subclover stunt virus promoters and terminators described in AU 689311.

The construct may produce a plant having reduced ability to produce fibre, fruit or seeds.

The present invention is suitable for the following applications :
A) Use of SuSy to enhance or otherwise improve fibre yield
   - SuSy may be used to enhance or otherwise improve fibre synthesis by increasing SuSy expression or activity in such plant cells e. g. a fibre specific or fibre enhanced promoter (such as but not limited to a cotton expansion promoter, or the promoter of the SuSy gene having the nucleotide sequence of SEQ ID NO : 1) or a constitutive promoter (such as CaMV 35S) can be used to express the SuSy gene in cotton cells.
   - Cotton fibre yield can be improved by reducing or suppressing cotton fuzz fibre. Fuzz fibre specific or enhanced promoter or constitutive promoter to reduce sucrose synthase selectively in cotton fuzz fibre cells. Reduced expression can be achieved by gene silencing technologies (antisense, cosuppression, dominant negative etc). Dominant negative mutant alleles of sucrose synthase genes may be obtained e. g. through mutation (insertion, substitution, or deletion of the phosphorylation site (s) of the sucrose synthase protein coding region)
   - Cotton fibre yield can be improved by modifying fuzz fibres into lint fibres e. g. by expression of SuSy coding region or a functional equivalent thereof by fibre specific or fibre enhanced promoter, primary or secondary cell wall promoter or a constitutive promoter.
B) Use of SuSy gene to improve fibre quality -Fibre length is an example of fibre quality. Expression of SuSy by a fibre specific or fibre enhanced promoter, primary cell wall promoter or a constitutive promoter.
C) Use of SuSy to improve fibre properties (e. g. fibre strength, length and number)
   - Fibre strength is an example of a fibre property. Fibre strength is significantly affected by the cellulose content in the secondary cell wall of fibre cells. This may be achieved by expression of SuSy by a fibre specific or fibre enhanced promoter, secondary cell wall promoter or a constitutive promoter.
D) Overexpression of SuSy specifically in seeds to increase sucrose utilisation in seeds for increased seed size and storage product content.
   - Cotton fibre yield can be improved by modifying fuzz fibres into lint fibres e.g. by expression of SuSy coding region or a functional equivalent thereof by fibre specific or fibre enhanced promoter, primary or secondary cell wall promoter or a constitutive promoter.
B) Use of SuSy gene to improve fibre quality
   - Fibre length is an example of fibre quality. Expression of SuSy by a fibre specific or fibre enhanced promoter, primary cell wall promoter or a constitutive promoter.
C) Use of SuSy to improve fibre properties (e.g. fibre strength, length and number)
   - Fibre strength is an example of a fibre property. Fibre strength is significantly effected by the cellulose content in the secondary cell wall of fibre cells. This may be achieved by expression of SuSy by a fibre specific or fibre enhanced promoter, secondary cell wall promoter or a constitutive promoter.
D) Overexpression of SuSy specifically in seeds to increase sucrose utilisation in seeds for increased seed size and storage product content.
E) Overexpression in the maternal tissue of a fruit (such as cotton fibre, horticultural fruit) for increased carbohydrate or fibre content.
F) Suppression of SuSy in seed with over expression of SuSy in fruit/fibre for increased fibre/fruit yield.

Preferred plant-operable promoters include the fibre specific and/or secondary cell wall specific promoters which can be isolated according to the teaching of WO 98/18949, WO98/00549 or US5932713.

The correlation of sucrose synthase activity in the ovule epidermis of cotton with fibre length also allows the use of sucrose synthase proteins (or antibodies or aptamers recognizing the same) or sucrose synthase coding regions as a diagnostic tool in cotton breeding. These may be used to identify cotton lines or varieties, including wild sources, which have enhanced sucrose synthase activity and increased potential to form longer fibres, particularly when crossed in a breeding program with other cotton lines and/or varieties having good fibre characteristics. Nucleic acids derived from sucrose synthase coding regions may be used to determine the amount of sucrose synthase RNA in the ovule epidermis. Also, polymorphism, including single nucleotide polymorphism between sucrose synthase genes may be used to identify lines with superior sucrose synthase alleles.

The methods and means of the invention are carried out in cotton plants, (e.g. both Gossypium hirsutum and Gossypium barbadense) for example Coker 312, Coker310, Coker5 AcalaSJ-5, GSC25110, FiberMax 819, Siokra 1-3, T25, GSA75, Acala SJ2, Acala SJ4, Acala SJ5, Acala SJ-C1, Acala B1644, Acala By 654-26, Acala B1654-43, Acala B3991, Acala GC356, Acala GC510, Acala GAM1, Acala C1, Acala Royale, Acala Maxxa, Acala Prema, Acala B638, Acala B1810, Acala B2724, Acala B4894, Acala B5002, non Acala "picker" Siokra, "stripper" variety FC2017, Coker 315, STONEVILLE 506, STONEVILLE 825, DP50, DP61, DP90, DP77, DES119, McN235, HBX87, HBX191, HBX107, FC 3027, CHEMBRED A1, CHEMBRED A2, CHEMBRED A3, CHEMBRED A4, CHEMBRED B1, CHEMBRED B2, CHEMBRED B3, CHEMBRED C1, CHEMBRED C2, CHEMBRED C3, CHEMBRED C4, PAYMASTER 145, HS26, HS46, SICALA, PIMA S6 and ORO BLANCO PIMA.

In order that the present invention may be more clearly understood preferred forms will be described with reference to the following examples and accompanying drawings.

### Modes for Carrying Out the Invention

### General molecular biology

Unless otherwise indicated, the recombinant DNA techniques utilised in the present invention are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (Editors), Current Protocols in Molecular Biology, Greene Pub. Associates and Wiley-Interscience (1988, including all updates until present) and are incorporated herein by reference.

Analysis for presence of the transgene by Southern blotting was carried out as described in Sambrook *et al.* (1989) and immunolocalisation of the SuSy protein was carried out on tissue sections as described in Ruan and Chourey (1998).

### Gene/DNA isolation

The DNA encoding a protein may be obtained from any cDNA library prepared from tissue or organisms believed to express the gene mRNA and to express it at a detectable level. The gene sequences can also be obtained from a genomic library or genomic DNA.

Libraries are screened with probes or analytical tools designed to identify the gene of interest or the protein encoded by it. For cDNA expression libraries, suitable probes include monoclonal or polyclonal antibodies that recognise and specifically bind the protein; oligonucleotides of about 20-80 bases in length that encode known or suspected portions of cDNA from the same or different species; and/or complementary or homologous cDNAs or fragments thereof that encode the same or a hybridising gene. Appropriate probes for screening genomic DNA libraries include, but are not limited to, oligonucleotides; cDNAs or fragments thereof that encode the same or hybridising DNA including expressed sequence tags and the like; and/or homologous genomic DNAs or fragments thereof. Screening the cDNA or genomic library with the selected probe may be conducted using standard procedures as described in chapters 10-12 of Sambrook et a/.

An alternative means to isolate a gene encoding is to use polymerase chain reaction (PCR) methodology as described in section 14 of Sambrook *et al*. This method requires the use of oligonucleotide probes that will hybridise to the gene.

The oligonucleotide sequences selected as probes should be of sufficient length and sufficiently unambiguous that false positives are minimised. The actual nucleotide sequence(s) is usually based on conserved or highly homologous nucleotide sequences or regions of the gene. The oligonucleotides may be degenerate at one or more positions. The use of degenerate oligonucleotides may be of particular importance where a library is screened from a species in which preferential codon usage in that species is known. The oligonucleotide must be labelled such that it can be detected upon hybridisation to DNA in the library being screened. The preferred method of labelling is to use ³²P-labelled dATP with polynucleotide kinase, as is well known in the art, to radiolabel the oligonucleotide. However, other methods may be used to label the oligonucleotide, including, but not limited to, biotinylation or enzyme labelling.

Nucleic acid having all the protein coding sequence is obtained by screening selected cDNA or genomic libraries, and if necessary, using conventional primer extension procedures as described in section 7.79 of Sambrook *et al.,* to detect precursors and processing intermediates of mRNA that may not have been reverse-transcribed into cDNA.

Another alternative method for obtaining the gene of interest is to chemically synthesise it using one of the methods described in Fingels et al. (Agnew Chem. Int. Ed. Engl. 28: 716-734, 1989). These methods include triester, phosphite, phosphoramidite and H-Phosphonate methods, PCR and other autoprimer methods, and oligonucleotide syntheses on solid supports. These methods may be used if the entire nucleic acid sequence of the gene is known, or the sequence of the nucleic acid complementary to the coding strand is available, or alternatively, if the target amino acid sequence is known, one may infer potential nucleic acid sequences using known and preferred coding residues for each amino acid residue.

### Cotton Transformation

Cotton was transformed using an Agrobacterium mediated transformation technique as described in F. Murray et al. 1999 (Molec. Breeding 5: 219-232). Transformed plants were grown in a glasshouse under natural illumination at day/night temperatures of approximately 30°C / 22°C.

### Example 1: Suppression of SuSy expression in cotton.

To provide evidence regarding the role of SuSy in fibre development, the present inventors transformed cotton with co-suppression and antisense SuSy constructs, targeting the 3' end of the seed SuSy cDNA driven by a constitutive subclover stunt seven virus promoter (see Figure 5 for a schematic representation). The presence of the transgene in 11 transgenic lines so far was confirmed by Southern analysis (see Figure 6). Among them two antisense lines (294-147, 292-43) and one co-suppression line (295-82) showed dramatic reductions in fibre and seed development with the remaining lines showing various degrees of inhibition of fibre growth.

Immunolocalization analysis on 0-d ovule sections (Figure 2) revealed that, compared to wild type fibre initials, SuSy protein was reduced to less than 20% wild type levels in fibres of line 294-147 and to undetectable levels in 295-82. Furthermore, the number and size of initiating fibres was reduced by at least 50% in these two lines as compared that in the wild type ovules. Indeed, by 6 days after anthesis (DAA), these transgenic seeds were virtually fibre-less (see Figure 1). Sucrose synthase activity was determined biochemically. Whereas WT plants show a SuSy activity of 22.5 nmol/min/seed, line 147 showed a SuSy activity of 5.8 nmol/min/seed and line 82 showed no detectable activity. This led to a dramatic reduction of normal fibre initial cells in the ovule epidermis. It is clear from these results that the degree of fibre initiation depends on the level of SuSy expression in the ovule epidermis.

The impact of SuSy expression on fibre initiation is further demonstrated by scanning electron microscopy analysis of the ovule epidermis of line 147 (see figure 7) revealed that many fibre cells are collapsed and shrunk (see figure 7B). Some fibre cells appeared, but these were much smaller in size, and were reduced in number when compared to wild type ovule (figure 7A). The shrunk and collapsed phenotype was clearly visible under high magnification (figure 7C).

These results demonstrate that some iso-forms of SuSy play a critical role in fibre initiation and elongation. Suppression of an iso-form of SuSy can thus be used to prevent or reduce fibre initiation and elongation while overexpression can thus be used to enhance fibre initiation and elongation.

While all the bolls in line 295-82 and 292-43 dropped off prematurely by 10 DAA, some bolls of line 294-147 were retained to maturity. In those mature bolls, most seeds were stunted, shrunken and fibre-less. About 15% of the seeds, however, showed 30% of the wild type fibre length (loose fibre as well) and wild type embryo and seed size, most likely due to segregation of the transgene.

This demonstrates that suppression of iso-forms of SuSy in the maternal tissue (seed coat/ fibre) alone can inhibit fibre development by ∼70% in length, while additional repression of SuSy in the embryo can arrest seed development entirely.

Gene suppression of iso-forms of SuSy in the embryo specifically to give a seedless phenotype (in a wide range of horticultural crops such as grape, peach, pear, apple).

Line 292-43 and 295-82 were male-sterile (no pollen) and the seed set can be recovered by pollination with wild-type pollen. This shows SuSy plays a role in male sterility of cotton.

Gene suppression of SuSy in male floral parts was found to cause sterility.
Sucrose synthase activity, the fresh weight of pollen and viability of the pollen were tested on developing anthers 2 days before flowering in different lines. The viability assay was conducted by staining with 2,3,5-triphenyltetrazolium chloride (TTC). The results are summarized in Table 1.

**Table 1. Suppression of SuSy in anthers leads to male sterility by pollen formation inhibition.**

| Line | SuSy activity (nmol min⁻¹ mg⁻¹) | Pollen fresh weight (% of WT pollen per anther) | Viable pollen (% of 100 grains) |
|---|---|---|---|
| WT | 44.1 ± 1.1 | 100.0 | 85.2 |
| 147#1 | 29.5 ± 6.9 | 30.0 | 52 |
| 147#2 | 14.7 ± 0.9 | 0.0 | 0.0 |
| 82 | 18.2 ± 1.5 | 0.0 | 0.0 |

A reduction of SuSy activity in anthers by about 33% results in an about 70% reduction of total pollen weight, largely due to the reduction of the number of pollen produced. Of the residual 30%, only half of the pollen was viable. When SuSy was reduced by about 60% no pollen was any longer formed. Pollen development is thus very sensitive to changes in SuSy activity. Pollen formation can thus be influenced (inhibited or promoted) by suppressing or overexpressing SuSy in anthers.

Suppression of SuSy activity in the maternal seed coat tissue in the transgenic cotton plants, also led to reduced fuzz length in the SuSy suppressed transgenic plants when compared to a wild type plant (see Figure 9). Fuzz fibre length was measured from the chalazal end of the mature seeds and the measurement was done by gently stretching the fuzz of seeds, delinted by hand, and measuring the length from the seed coat epidermis to the tip. The brown color visible in the transgenic line, is due to the fact that fuzz is shorter in transgenic lines than in WT, where the brown seed coat is well covered by relatively long fuzz. SuSy activity was also determined. The results can be summarized as follows:
- the wild type plant had a fuzz length of 2.2 mm (and a SuSy activity measurement set at 100%)
- transgenic line 147#2 had a fuzz length of 1.4 mm (and a SuSy activity measurement of 36% of the WT).

From the general correlation detected between SuSy expression and fibre content and/or quality described herein, it will be understood that overexpression of SuSy gene will have potential beneficial effects on fibre, fruit and seed production in plants.

Example 2. Analysis of progeny of cotton lines 82, 147 and 43 with suppressed SuSy activity.
To confirm the correlation between the presence of the SuSy suppression transgene and the reduced fibre initiation in cotton, further analysis was conducted on the T1 generation. About 24 T1 seeds were sown from 6 T0 lines. All germinated seedlings were screened by PCR for presence or absence of the transgenes (nptII; S7 promoter and SuSy suppression transgene). Six PCR positive and one PCR negative segregating individuals from each of the 6 lines were further analysed on molecular biochemical and cellular level. Line 82 was propagated vegetatively as a reference point. The fibre-less phenotype in the different lines of the T0 generation (indicated by arrows in figure 6A) was still preserved in respectively 3 and 2 segregating individuals of the T1 generation for line 147 and 43 respectively (arrows in Figure 6B). The remaining individuals show various degree of fibre/ seed suppression. The vegetatively-kept line 82 continues to produced fibreless seeds. These results demonstrated that the fibreless/ or seed suppressed phenotype described was indeed due to the presence of SuSy transgene.

A positive strong correlation could be found between the degree of sucrose activity in the seed epidermis and the fibre length in segregating T1 generation plants of lines 147, 43 and 101 (see Figures 8, 9 and 10). When fibre length was plotted against sucrose synthase activity for the different plants of the different lines as well as the wild type line, a linear regression could be derived (Y=2.634X-50; R²=0.98). The linear nature of the correlation up to the wild-type level, indicates that overexpression of the sucrose synthase activity above wild type level will lead to fibre length greater than fibre length in wild type plants.

A similar strong correlation exists between the reduction in SuSy activity and mature cotton fibre length and dry weight. SuSy activity in seed coat epidermis has been reduced to 28.8%, 42.5% and 53.9% of the wild type level in segregating individuals #1, #2 and #3 of line 43 T1 generation plants respectively. Lint fibre dry weight per boll was 11.1%, 51.6%, and 70.9% respectively.

Cellulose content in cotton fibre is also closely correlated to the level of SuSy expression. Cellulose content was analysed by labelling cotton fibre at day 25 (peak of cellulose synthesis in vivo) with Calcofluor white, a fluorescent dye specifically binding to cellulose. The fluorescent intensity of the labelled fibres, indicating cellulose content, was significantly reduced in the SuSy suppressed transgenic lines when compared to fluorescence in the wild type. The degree of fluorescence reduction correlated to the degree of suppression of SuSy activity.

### Example 3. Overexpression of sucrose synthase in cotton plants.

The coding sequence of a potato sucrose synthase cDNA (Genbank Accession number M18745) is operably linked to a subterranean clover stunt virus promoter (S7; WO9606932) and a 3' transcription termination and polyadenylation signal functional in plants. This chimeric gene is operably linked to a selectable marker gene and introduced into a T-DNA vector. Cotton plants are transformed using the above mentioned Agrobacterium mediated transformation technique. Transgenic cotton lines are identified, sucrose synthase activity, fibre length, fuzz fibre length, cellulose content, and dry weight of the lint is analyzed. A positive correlation is found between SuSy activity and increased fibre length cellulose content, and dry weight of the lint.

### SUMMARY OF THE EXAMPLES

The single-cell cotton fibres initiate from ovule epidermis at anthesis, elongate to 2.5 ∼ 3.0 cm in about 16 days and then synthesize massive amounts of cellulose. Thus, cotton fibre is an excellent system for the study of cell differentiation, elongation and cellulose synthesis in higher plants with significant industry implications for improving fibre yield and quality.

To provide definitive evidence regarding the role of SuSy in fibre and seed development, the present inventors transformed cotton with co-suppression and antisense SuSy constructs, targeting the 3' end of the seed SuSy cDNA driven by subclover stunt seven virus promoter. The presence of the transgene in 9 transgenic lines so far was confirmed by Southern analysis. Among them one antisense line (294-147) and one co-suppression line (295-82) showed dramatic reductions in fibre and seed development with the remaining lines showing various degrees of inhibition of fibre growth. Immunolocalization analysis on 0-d ovule sections revealed that, compared to wild type fibre initials, SuSy protein was reduced to only about 20% wild type levels in fibres of line 294-147 and to undetectable levels in 295-82. Furthermore, the number and size of initiating fibres was reduced by at least 50% in these two lines as compared that in the wild type ovules. Indeed, by 2 d after anthesis (DAA), these transgenic seeds were virtually fibre-less. This is in contrast to a fibre-covered seed phenotype seen in the wild type plants at this stage. While all the bolls in line 295-82 dropped off prematurely by 10 DAA, some bolls of line 294-147 were retained to maturity. In those mature bolls, most seeds were stunted, shrunken and fibre-less. About 15% of the seeds, however, showed 30% of the wild type fibre length and wild type embryo and seed size, most likely due to segregation of the transgene. These results demonstrate (a) that SuSy plays a critical role in fibre initiation and elongation, (b) that suppression of SuSy in the maternal tissue (seed coat/fibre) alone can inhibit fibre development while additional repression of SuSy in the embryo can arrest seed development entirely. The influence of SuSy suppression on the reduction of fibre length has been confirmed by analysis of progeny of the transgenic cotton lines. Further, a linear correlation was found between the level of SuSy activity and the increase in fibre length. The linear nature of this correlation indicates that in wild type cotton, the level of sucrose synthase is limiting.

Overexpression of sucrose synthase at least in maternal ovule tissue increases fibre length in fibre producing plants.

### References:

Amor, Y., Haigler, C.H., Johnson, S., Wainscott, M. and Delmer, D.P. (1995) A membrane associated form of sucrose synthase and its potential role in synthesis of cellulose and callose in plants. Proc Nat Acad Science USA 92, 9353-9357.
Ruan, Y.-L., Llewellyn, D.J, Furbank, R.T. (2000) Pathway and control of sucrose import into initiating fibre cells. Australian Journal of Plant Physiology 27, 795-800.
Ruan, Y.-L. and Chourey, P.S. (1998) A fibreless seed (fls) mutation in cotton is associated with lack of fibre cell initiation in ovule epidermis, alteration in sucrose synthase expression and carbon partitioning in developing seed. Plant Physiology 118: 399-406
Ruan, Y.-L., Chourey, P.S., Delmer, D.P. and Luis, P.G. (1997) The differential expression of sucrose synthase in relation to diverse patterns of carbon partitioning in developing cotton seed. Plant Physiology 115: 375-385

## Claims

1. A method for increasing fibre length in a cotton plant comprising providing cells of said cotton plant with a chimeric gene comprising the following operably linked DNA fragments:
(i) a plant operable promoter;
(ii) a coding region which when transcribed yields an RNA said RNA being capable of being translated into an active sucrose synthase protein; and
(iii) a transcription termination and polyadenylation signal that functions in said plant cells.

2. The method according to claim 1, wherein said coding region comprises a nucleotide sequence selected from the group consisting of:
(a) a nucleotide sequence encoding a polypeptide comprising the amino acid sequence of SEQ ID NO: 2 or a fragment of said nucleotide sequence encoding an active sucrose synthase;
(b) the nucleotide sequence of SEQ ID NO: 1 or a fragment of said nucleotide sequence encoding an active sucrose synthase;
(c) a nucleotide sequence having at least 70% sequence identity to (a) or (b) encoding an active sucrose synthase; and
(d) a nucleotide sequence that hybridizes under stringent conditions with a complementary sequence of (a) or (b) or a part thereof encoding an active sucrose synthase.

3. The method according to claim 1 or 2, wherein said promoter is selected from a subterranean clover stunt virus promoter, a constitutive promoter, a CaMV35S promoter, a fibre specific or fibre enhanced promoter or a secondary cell wall specific promoter.

4. A cotton plant comprising in its genome a chimeric DNA comprising the following operably linked DNA fragments:
(i) a plant operable promoter;
(ii) a coding region which when transcribed yields an RNA said RNA being capable of being translated into an active sucrose synthase protein; and
(iii) a transcription termination and polyadenylation signal that functions in said plant cells.

5. The cotton plant of claim 4 wherein the active sucrose synthase protein is expressed in fibre cells and wherein said fibre cells have an increased sucrose synthase activity compared to fibre cells of plant cells which do not comprise said chimeric DNA.

6. The cotton plant of claim 4 or 5 wherein the fibre cells are fibre initial cells.

7. The cotton plant according to any one of claims 4 to 6, wherein said chimeric DNA comprises a coding region as defined in claim 2.

8. Seeds of the plant according to any one of claims 4 to 7, wherein said seed comprises a chimeric gene as defined in any one of claims 1, 2 or 3.

## Patentansprüche

1. Verfahren zum Erhöhen der Faserlänge in einer Baumwollpflanze, bei dem man Zellen der Baumwollpflanze mit einem chimären Gen ausstattet, das die folgenden funktionsfähig verbundenen DNA-Fragmente umfasst :
(i) einen in Pflanzen funktionsfähigen Promotor,
(ii) eine codierende Region, die nach Transkription eine RNA ergibt, wobei die RNA in ein aktives Saccharosesynthase-Protein translatiert werden kann, und
(iii) ein Transkriptionsterminations- und Polyadenylierungssignal, das in den Pflanzenzellen funktioniert.

2. Verfahren nach Anspruch 1, wobei die codierende Region eine Nukleotidsequenz umfasst, die aus der Gruppe ausgewählt wird, bestehend aus :
(a) einer Nukleotidsequenz, die ein Polypeptid codiert, das die Aminosäuresequenz SEQ ID NR: 2 umfasst, oder einem Fragment der Nukleotidsequenz, das eine aktive Saccharosesynthase codiert,
(b) der Nukleotidsequenz der SEQ ID NR: 1 oder einem Fragment der Nukleotidsequenz, das eine aktive Saccharosesynthase codiert,
(c) einer Nukleotidsequenz mit mindestens 70% Identität zu (a) oder (b), die eine aktive Saccharosesynthase codiert, und
(d) einer Nukleotidsequenz, die unter stringenten Bedingungen an eine komplementäre Sequenz von (a) oder (b) oder einen Teil davon, der eine aktive Saccharosesynthase codiert, hybridisiert.

3. Verfahren nach Anspruch 1 oder 2, wobei der Promotor aus einem Subterranean Clover Stunt-Virus-Promotor, einem konstitutiven Promotor, einem CaMV35S-Promotor, einem faserspezifischen oder in Fasern verstärkten Promotor oder einem spezifischen Promotor der sekundären Zellwand ausgewählt wird.

4. Baumwollpflanze, die in ihrem Genom eine chimäre DNA umfasst, die die folgenden funktionsfähig verbundenen DNA-Fragmente umfasst :
(i) einen in Pflanzen funktionsfähigen Promotor,
(ii) eine codierende Region, die nach Transkription eine RNA ergibt, wobei die RNA in ein aktives Saccharosesynthase-Protein translatiert werden kann, und
(iii) ein Transkriptionsterminations- und Polyadenylierungssignal, das in den Pflanzenzellen funktioniert.

5. Baumwollpflanze nach Anspruch 4, wobei das aktive Saccharosesynthase-Protein in Faserzellen exprimiert wird und wobei die Faserzellen eine erhöhte Saccharosesynthase-Aktivität im Vergleich zu Faserzellen von Pflanzenzellen, die nicht die chimäre DNA umfassen, besitzen.

6. Baumwollpflanze nach Anspruch 4 oder 5, wobei die Faserzellen Faser-Initialzellen sind.

7. Baumwollpflanze nach einem der Ansprüche 4 bis 6, wobei die chimäre DNA eine codierende Region nach Anspruch 2 umfasst.

8. Samen der Pflanze nach einem der Ansprüche 4 bis 7, wobei der Samen ein chimäres Gen nach einem der Ansprüche 1, 2 oder 3 umfasst.

## Revendications

1. Procédé destiné à augmenter la longueur des fibres dans un plant de coton, comprenant l'étape consistant à fournir à des cellules dudit plant de coton un gène chimère comprenant les fragments d'ADN suivants étant liés de manière opérationnelle :
(i) un promoteur végétal opérationnel ;
(ii) une région codante qui, quand elle est transcrite, produit un ARN, ledit ARN étant capable d'être traduit en une protéine active de saccharose synthase ; et
(iii) un signal de terminaison de la transcription et de polyadénylation qui fonctionne dans lesdites cellules végétales.

2. Procédé selon la revendication 1, dans lequel ladite région codante comprend une séquence nucléotidique choisie dans le groupe constitué par :
(a) une séquence nucléotidique codant pour un polypeptide, qui comprend la séquence d'acides aminés de SEQ ID n° : 2, ou un fragment de ladite séquence nucléotidique codant pour une saccharose synthase active ;
(b) la séquence nucléotidique de SEQ ID n° : 1 ou un fragment de ladite séquence nucléotidique codant pour une saccharose synthase active ;
(c) une séquence nucléotidique ayant une identité de séquence d'au moins 70% avec les (a) ou (b), qui codent pour une saccharose synthase active ; et
(d) une séquence nucléotidique qui s'hybride, dans des conditions stringentes, avec une séquence complémentaire des (a) ou (b) ou une partie de celle-ci codant pour une saccharose synthase active.

3. Procédé selon la revendication 1 ou la 2, dans lequel ledit promoteur est choisi parmi un promoteur du virus du rabougrissement du trèfle souterrain, un promoteur constitutif, un promoteur de CaMV 35S, un promoteur spécifique des fibres ou amplifié dans les fibres, ou bien un promoteur spécifique de la paroi cellulaire secondaire.

4. Plant de coton comprenant dans son génome un ADN chimère, qui comprend les fragments d'ADN suivants étant liés de manière opérationnelle :
(i) un promoteur végétal opérationnel ;
(ii) une région codante qui, quand elle est transcrite, produit un ARN, ledit ARN étant capable d'être traduit en une protéine active de saccharose synthase ; et
(iii) un signal de terminaison de la transcription et de polyadénylation qui fonctionne dans lesdites cellules végétales.

5. Plant de coton selon la revendication 4, dans lequel la protéine active de saccharose synthase est exprimée dans les cellules des fibres et dans lequel lesdites cellules des fibres ont une activité accrue de saccharose synthase, en comparaison avec les cellules des fibres de cellules végétales qui ne comprennent pas ledit ADN chimère.

6. Plant de coton selon la revendication 4 ou la 5, dans lequel les cellules des fibres sont des cellules initiales des fibres.

7. Plant de coton selon l'une quelconque des revendications 4 à 6, dans lequel ledit ADN chimère comprend une région codante telle que définie dans la revendication 2.

8. Graines de la plante selon l'une quelconque des revendications 4 à 7, dans laquelle ladite graine comprend un gène chimère tel que défini dans l'une quelconque des revendications 1, 2 ou 3.


### SEQUENCE LISTING

<110> Commonwealth Scientific Industrial and Research Organisation
<120> Modification of sucrose synthase gene expression in plant tissue and uses therefor
<130> 500159/MRO
<150> PR1975
   <151> 2000-12-08
<150> US60/251852
   <151> 2000-12-08
<160> 2
<170> PatentIn version 3.1
<210> 1
   <211> 2625
   <212> DNA
   <213> Gossypium hirsutum
<220>
   <221> misc_feature
   <222> (1240)..(1240)
   <223> N is any nucleotide
<220>
   <221> exon
   <222> (1)..(2418)
   <223>
<400> 1
   atg gct gag cgt gct ctc act cgc gtc cac agt ctc cgt gag cgt ttg      48
   Met ala Glu Arg Ala Leu Thr Arg Val His Ser Leu Arg Glu Arg Leu
   1               5                   10                  15
   gat gag acc ctt ctt gct cac agg aac gag att ttg gcc ttg ctc tca      96
   Asp Glu Thr Leu Leu Ala His Arg Asn Glu Ile Leu Ala Leu Leu Ser
               20                  25                  30
   agg atc gag ggc aaa gga aaa gga att ctg caa cac cat caa att att     144
   Arg Ile Glu Gly Lys Gly Lys Gly Ile Leu Gln His His Gln Ile Ile
           35                  40                  45
   cta gag ttt gaa gct atc cct gaa gag aac aga aag aag ctc gct aat     192
   Leu Glu Phe Glu Ala Ile Pro Glu Glu Asn Arg Lys Lys Leu Ala Asn
       50                  55                  60
   ggt gca ttt ttt gaa gta ttg aag gct agt cag gaa gcg atc gtg ttg     240
   Gly Ala Phe Phe Glu Val Leu Lys Ala Ser Gln Glu Ala Ile Val Leu
   65                  70                  75                  80
   cct cca tgg gtt gca ctt gct gtt cgt cca agg cct ggt gtt tgg gag     288
   Pro Pro Trp Val Ala Leu Ala Val Arg Pro Arg Pro Gly Val Trp Glu
                   85                  90                  95
   tac att aga gtg aat gtt cac gcc ctt gtt gtt gag gaa ctc act gtt     336
   Tyr Ile Arg Val Asn Val His Ala Leu Val Val Glu Glu Leu Thr Val
               100                 105                 110
   gct gag tat ctc cac ttc aag gaa gag ctt gtt gat gga agt tca aat      384
   Ala Glu Tyr Leu His Phe Lys Glu Glu Leu Val Asp Gly Ser Ser Asn
           115                 120                 125
   gga aac ttt gtt ttg gaa ttg gat ttt gag ccc ttc aac tca tca ttc      432
   Gly Asn Phe Val Leu Glu Leu Asp Phe Glu Pro Phe Asn Ser Ser Phe
       130                 135                 140
   ccc cgc cca act ctt tca aaa tcc att ggt aat ggt gtg gag ttc cta      480
   Pro Arg Pro Thr Leu Ser Lys Ser Ile Gly Asn Gly Val Glu Phe Leu
   145                 150                 155                 160
   aat cgt cac ctt tcg gca aaa ttg ttc cat gac aag gag agc atg cac      528
   Asn Arg His Leu Ser Ala Lys Leu Phe His Asp Lys Glu Ser Met His
                   165                 170                 175
   cct ttg ctc gaa ttc ctc aga gtc cat tgt cac aag ggc aag aac atg      576
   Pro Leu Leu Glu Phe Leu Arg Val His Cys His Lys Gly Lys Asn Met
               180                 185                 190
   atg ttg aat gac aga att cag aac ttg aat gct ctt caa cat gtt ttg      624
   Met Leu Asn Asp Arg Ile Gln Asn Leu Asn Ala Leu Gln His Val Leu
           195                 200                 205
   agg aaa gca gag gag tat ctt ggt acc cta cct cct gag aca cca tgt      672
   Arg Lys Ala Glu Glu Tyr Leu Gly Thr Leu Pro Pro Glu Thr Pro Cys
       210                 215                 220
   gcc gaa ttc gaa cac cgg ttc cag gaa atc ggt ttg gaa aga ggt tgg      720
   Ala Glu Phe Glu His Arg Phe Gln Glu Ile Gly Leu Glu Arg Gly Trp
   225                 230                 235                 240
   ggt gac acc gca gaa cgc gtg ctc gag atg atc caa ctc ctt ttg gat      768
   Gly Asp Thr Ala Glu Arg Val Leu Glu Met Ile Gln Leu Leu Leu Asp
                   245                 250                 255
   ctt ctt gag gca act gat cct tgc acc ctt gag aag ttc ctt ggg aga      816
   Leu Leu Glu Ala Thr Asp Pro Cys Thr Leu Glu Lys Phe Leu Gly Arg
               260                 265                 270
   atc ccc atg gtg ttc aat gtt gtg att ctc act ccc cac gga tac ttc      864
   Ile Pro Met Val Phe Asn Val Val Ile Leu Thr Pro His Gly Tyr Phe
           275                 280                 285
   gct caa gac aat gtt ttg ggg tat ccc gac acc ggt ggg cag gtt gtt      912
   Ala Gln Asp Asn Val Leu Gly Tyr Pro Asp Thr Gly Gly Gln Val Val
       290                 295                 300
   tac atc ttg gat caa gtc cga gct ttg gag aat gag atg ctc ctc cgt      960
   Tyr Ile Leu Asp Gln Val Arg Ala Leu Glu Asn Glu Met Leu Leu Arg
   305                 310                 315                 320
   ata aag caa caa gga ctc aac atc acc cct cga atc ctc att att act     1008
   Ile Lys Gln Gln Gly Leu Asn Ile Thr Pro Arg Ile Leu Ile Ile Thr
                   325                 330                 335
   aga ctt ctt cct gat gct gtc gga aca aca tgc ggt caa cga ctt gag     1056
   Arg Leu Leu Pro Asp Ala Val Gly Thr Thr Cys Gly Gln Arg Leu Glu
               340                 345                 350
   aaa gta tac gga aca gag cac tcg gat att ctt cga gta ccc ttc aga     1104
   Lys Val Tyr Gly Thr Glu His Ser Asp Ile Leu Arg Val Pro Phe Arg
           355                 360                 365
   aca gaa aag gga att gtt cga aaa tgg atc tca aga ttt gaa aaa gtc      1152
   Thr Glu Lys Gly Ile Val Arg Lys Trp Ile Ser Arg Phe Glu Lys Val
       370                 375                 380
   tgg cca tac ttg gaa acc tac aca gag gat gtt gct cat gaa atc tcc      1200
   Trp Pro Tyr Leu Glu Thr Tyr Thr Glu Asp Val Ala His Glu Ile Ser
   385                 390                 395                 400
   aaa gag ttg cac ggc acg cca gat ctg atc atc gga aac nac agc gac      1248
   Lys Glu Leu His Gly Thr Pro Asp Leu Ile Ile Gly Asn Xaa Ser Asp
                   405                 410                 415
   ggc aat atc gtc gcc tcc ttg ctc gca cat aaa tta ggt gtc aca cag      1296
   Gly Asn Ile Val Ala Ser Leu Leu Ala His Lys Leu Gly Val Thr Gln
               420                 425                 430
   tgc acc atc gcc cat gct ttg gag aag aca aaa tat cca gat tca gat      1344
   Cys Thr Ile Ala His Ala Leu Glu Lys Thr Lys Tyr Pro Asp Ser Asp
           435                 440                 445
   atc tat tgg aag aag ctt gaa gac aaa tac cat ttc tct tgc caa ttt      1392
   Ile Tyr Trp Lys Lys Leu Glu Asp Lys Tyr His Phe Ser Cys Gln Phe
       450                 455                 460
   aca gct gat ctt ttt gca atg aac cat aca gat ttc atc atc acc agt      1440
   Thr Ala Asp Leu Phe Ala Met Asn His Thr Asp Phe Ile Ile Thr Ser
   465                 470                 475                 480
   act ttc cag gaa att gca gga agc aag gac act gtt ggt caa tac gag      1488
   Thr Phe Gln Glu Ile Ala Gly Ser Lys Asp Thr Val Gly Gln Tyr Glu
                   485                 490                 495
   agc cac act gct ttc act ctt cct ggt ctc tac cgt gtt gta cat ggt      1536
   Ser His Thr Ala Phe Thr Leu Pro Gly Leu Tyr Arg Val Val His Gly
               500                 505                 510
   atc gat gtg ttt gat ccc aaa ttc aac att gtt tcc cct ggt gct gat      1584
   Ile Asp Val Phe Asp Pro Lys Phe Asn Ile Val Ser Pro Gly Ala Asp
           515                 520                 525
   atg gag ata tac ttc cct tac acc gaa gag aag cgg agg ttg aag cat      1632
   Met Glu Ile Tyr Phe Pro Tyr Thr Glu Glu Lys Arg Arg Leu Lys His
       530                 535                 540
   ttc cat cct gag atc gaa gac ctt ctt tac acc aaa gtt gag aat gaa      1680
   Phe His Pro Glu Ile Glu Asp Leu Leu Tyr Thr Lys Val Glu Asn Glu
   545                 550                 555                 560
   gaa cac tta tgt gtg ctc aat gac cgc aac aag cca att ctg ttc aca      1728
   Glu His Leu Cys Val Leu Asn Asp Arg Asn Lys Pro Ile Leu Phe Thr
                   565                 570                 575
   atg cca agg ctt gat cgt gtc aag aac tta acc gga ctc gtc gag tgg      1776
   Met Pro Arg Leu Asp Arg Val Lys Asn Leu Thr Gly Leu Val Glu Trp
               580                 585                 590
   tgc ggc aag aac cca aag ttg cgt gag ttg gct aac ctc gta gtt gta      1824
   Cys Gly Lys Asn Pro Lys Leu Arg Glu Leu Ala Asn Leu Val Val Val
           595                 600                 605
   ggt ggt gat agg cga aag gaa tct aaa gat ttg gaa gag aag gct gaa      1872
   Gly Gly Asp Arg Arg Lys Glu Ser Lys Asp Leu Glu Glu Lys Ala Glu
       610                 615                 620
   atg aag aaa atg ttt gag ctg atc gac aag tac aac ttg aac ggc caa      1920
   Met Lys Lys Met Phe Glu Leu Ile Asp Lys Tyr Asn Leu Asn Gly Gln
   625                 630                 635                 640
   ttc aga tgg ata tca tct caa atg aac aga atc cga aat gtt gaa ctt      1968
   Phe Arg Trp Ile Ser Ser Gln Met Asn Arg Ile Arg Asn Val Glu Leu
                   645                 650                 655
   tac cga tac att tgc gac acg aaa ggt gcc ttt gta cag cct gca ttg      2016
   Tyr Arg Tyr Ile Cys Asp Thr Lys Gly Ala Phe Val Gln Pro Ala Leu
               660                 665                 670
   tat gaa gcc ttt gga ttg aca gtt gtg gag gca atg act tgc ggt ttg      2064
   Tyr Glu Ala Phe Gly Leu Thr Val Val Glu Ala Met Thr Cys Gly Leu
           675                 680                 685
   cca aca ttc gca acc tgt aac ggt gga cca gcc gag att att gtc cat      2112
   Pro Thr Phe Ala Thr Cys Asn Gly Gly Pro Ala Glu Ile Ile Val His
       690                 695                 700
   ggg aaa tct ggt ttc aac att gat cct tac cat ggt gat caa gct gct      2160
   Gly Lys Ser Gly Phe Asn Ile Asp Pro Tyr His Gly Asp Gln Ala Ala
   705                 710                 715                 720
   gac ata ctc gtc gat ttc ttt gaa aag tgt aag aaa gat cca tct cac      2208
   Asp Ile Leu Val Asp Phe Phe Glu Lys Cys Lys Lys Asp Pro Ser His
                   725                 730                 735
   tgg gat aag atc tcc caa gga ggc ttg aaa cga ata gag gag aag tat      2256
   Trp Asp Lys Ile Ser Gln Gly Gly Leu Lys Arg Ile Glu Glu Lys Tyr
               740                 745                 750
   aca tgg aag att tac tcg gag aga cta ttg acc ctg aca gga gtg tat      2304
   Thr Trp Lys Ile Tyr Ser Glu Arg Leu Leu Thr Leu Thr Gly Val Tyr
           755                 760                 765
   gga ttc tgg aag cat gtt tcc aac ctt gaa cgc cgt gag agt cgt cgt      2352
   Gly Phe Trp Lys His Val Ser Asn Leu Glu Arg Arg Glu Ser Arg Arg
       770                 775                 780
   tac ctt gag atg ttt tat gct ctt aag tac cgt aag ctg gct gaa tca      2400
   Tyr Leu Glu Met Phe Tyr Ala Leu Lys Tyr Arg Lys Leu Ala Glu Ser
   785                 790                 795                 800
   gtt cca ttg gca gag gag taaattgaac ctgttaaata acattgggcc             2448
   Val Pro Leu Ala Glu Glu
                   805
   ggtttttctt ggagaataat attctgtttt gtaatttcaa ttggagaagc tcctttgtat    2508
   ttcatcttgt cttttccttt tccttttttc gccggcattg tttgaacatg gggttgtgcg     2568
   cccgtcaatt ccagttaaat atggtgactt ttgtttttca aaaaaaaaaa aaaaaaa       2625
<210> 2
   <211> 806
   <212> PRT
   <213> Gossypium hirsutum
<220>
   <221> misc_feature
   <222> (414)..(414)
   <223> Xaa is Asn, Asp, His, or Tyr
<400> 2
   Met ala Glu Arg Ala Leu Thr Arg Val His Ser Leu Arg Glu Arg Leu
   1               5                   10                  15
   Asp Glu Thr Leu Leu Ala His Arg Asn Glu Ile Leu Ala Leu Leu Ser
               20                  25                  30
   Arg Ile Glu Gly Lys Gly Lys Gly Ile Leu Gln His His Gln Ile Ile
           35                  40                  45
   Leu Glu Phe Glu Ala Ile Pro Glu Glu Asn Arg Lys Lys Leu Ala Asn
       50                  55                  60
   Gly Ala Phe Phe Glu Val Leu Lys Ala Ser Gln Glu Ala Ile Val Leu
   65                  70                  75                  80
   Pro Pro Trp Val Ala Leu Ala Val Arg Pro Arg Pro Gly Val Trp Glu
                   85                  90                  95
   Tyr Ile Arg Val Asn Val His Ala Leu Val Val Glu Glu Leu Thr Val
               100                 105                 110
   Ala Glu Tyr Leu His Phe Lys Glu Glu Leu Val Asp Gly Ser Ser Asn
           115                 120                 125
   Gly Asn Phe Val Leu Glu Leu Asp Phe Glu Pro Phe Asn Ser Ser Phe
       130                 135                 140
   Pro Arg Pro Thr Leu Ser Lys Ser Ile Gly Asn Gly Val Glu Phe Leu
   145                 150                 155                 160
   Asn Arg His Leu Ser Ala Lys Leu Phe His Asp Lys Glu Ser Met His
                   165                 170                 175
   Pro Leu Leu Glu Phe Leu Arg Val His Cys His Lys Gly Lys Asn Met
               180                 185                 190
   Met Leu Asn Asp Arg Ile Gln Asn Leu Asn Ala Leu Gln His Val Leu
           195                 200                 205
   Arg Lys Ala Glu Glu Tyr Leu Gly Thr Leu Pro Pro Glu Thr Pro Cys
       210                 215                 220
   Ala Glu Phe Glu His Arg Phe Gln Glu Ile Gly Leu Glu Arg Gly Trp
   225 230 235 240
   Gly Asp Thr Ala Glu Arg Val Leu Glu Met Ile Gln Leu Leu Leu Asp
   245 250 255
   Leu Leu Glu Ala Thr Asp Pro Cys Thr Leu Glu Lys Phe Leu Gly Arg
   260 265 270
   Ile Pro Met Val Phe Asn Val Val Ile Leu Thr Pro His Gly Tyr Phe
   275 280 285
   Ala Gln Asp Asn Val Leu Gly Tyr Pro Asp Thr Gly Gly Gln Val Val
   290 295 300
   Tyr Ile Leu Asp Gln Val Arg Ala Leu Glu Asn Glu Met Leu Leu Arg
   305 310 315 320
   Ile Lys Gln Gln Gly Leu Asn Ile Thr Pro Arg Ile Leu Ile Ile Thr
   325 330 335
   Arg Leu Leu Pro Asp Ala Val Gly Thr Thr Cys Gly Gln Arg Leu Glu
   340 345 350
   Lys Val Tyr Gly Thr Glu His Ser Asp Ile Leu Arg Val Pro Phe Arg
   355 360 365
   Thr Glu Lys Gly Ile Val Arg Lys Trp Ile Ser Arg Phe Glu Lys Val
   370 375 380
   Trp Pro Tyr Leu Glu Thr Tyr Thr Glu Asp Val Ala His Glu Ile Ser
   385 390 395 400
   Lys Glu Leu His Gly Thr Pro Asp Leu Ile Ile Gly Asn Xaa Ser Asp
   405 410 415
   Gly Asn Ile Val Ala Ser Leu Leu Ala His Lys Leu Gly Val Thr Gln
   420 425 430
   Cys Thr Ile Ala His Ala Leu Glu Lys Thr Lys Tyr Pro Asp Ser Asp
   435 440 445
   Ile Tyr Trp Lys Lys Leu Glu Asp Lys Tyr His Phe Ser Cys Gln Phe
   450 455 460
   Thr Ala Asp Leu Phe Ala Met Asn His Thr Asp Phe Ile Ile Thr Ser
   465 470 475 480
   Thr Phe Gln Glu Ile Ala Gly Ser Lys Asp Thr Val Gly Gln Tyr Glu
   485 490 495
   Ser His Thr Ala Phe Thr Leu Pro Gly Leu Tyr Arg Val Val His Gly
   500 505 510
   Ile Asp Val Phe Asp Pro Lys Phe Asn Ile Val Ser Pro Gly Ala Asp
   515 520 525
   Met Glu Ile Tyr Phe Pro Tyr Thr Glu Glu Lys Arg Arg Leu Lys His
   530 535 540
   Phe His Pro Glu Ile Glu Asp Leu Leu Tyr Thr Lys Val Glu Asn Glu
   545 550 555 560
   Glu His Leu Cys Val Leu Asn Asp Arg Asn Lys Pro Ile Leu Phe Thr
   565 570 575
   Met Pro Arg Leu Asp Arg Val Lys Asn Leu Thr Gly Leu Val Glu Trp
   580 585 590
   Cys Gly Lys Asn Pro Lys Leu Arg Glu Leu Ala Asn Leu Val Val Val
   595 600 605
   Gly Gly Asp Arg Arg Lys Glu Ser Lys Asp Leu Glu Glu Lys Ala Glu
   610 615 620
   Met Lys Lys Met Phe Glu Leu Ile Asp Lys Tyr Asn Leu Asn Gly Gln
   625 630 635 640
   Phe Arg Trp Ile Ser Ser Gln Met Asn Arg Ile Arg Asn Val Glu Leu
   645 650 655
   Tyr Arg Tyr Ile Cys Asp Thr Lys Gly Ala Phe Val Gln Pro Ala Leu
   660 665 670
   Tyr Glu Ala Phe Gly Leu Thr Val Val Glu Ala Met Thr Cys Gly Leu
   675 680 685
   Pro Thr Phe Ala Thr Cys Asn Gly Gly Pro Ala Glu Ile Ile Val His
   690 695 700
   Gly Lys Ser Gly Phe Asn Ile Asp Pro Tyr His Gly Asp Gln Ala Ala
   705 710 715 720
   Asp Ile Leu Val Asp Phe Phe Glu Lys Cys Lys Lys Asp Pro Ser His
   725 730 735
   Trp Asp Lys Ile Ser Gln Gly Gly Leu Lys Arg Ile Glu Glu Lys Tyr
   740 745 750
   Thr Trp Lys Ile Tyr Ser Glu Arg Leu Leu Thr Leu Thr Gly Val Tyr
   755 760 765
   Gly Phe Trp Lys His Val Ser Asn Leu Glu Arg Arg Glu Ser Arg Arg
   770 775 780
   Tyr Leu Glu Met Phe Tyr Ala Leu Lys Tyr Arg Lys Leu Ala Glu Ser
   785 790 795 800
   Val Pro Leu Ala Glu Glu
   805